# EUROPÄISCHE PATENTSCHRIFT

(11) **EP 1 730 291 B1**
(45) Veröffentlichungstag und Bekanntmachung des Hinweises auf die Patenterteilung: **05.12.2012**
(21) Anmeldenummer: 05715626.7
(22) Anmeldetag: 01.03.2005
(51) Int. Cl.: C12P 13/02, C12N 9/78

(54) **NITRILHYDRATASE AUS RHODOCOCCUS**
NITRILE HYDRATASE OF RHODOCOCCUS
NITRILHYDRATASE ISSUE DE RHODOCOCCUS

(30) Priorität: 20.03.2004 DE 102004013824
(43) Veröffentlichungstag der Anmeldung: 13.12.2006
(73) Patentinhaber: Evonik Degussa GmbH, 45128 Essen (DE)
(72) Erfinder: OSSWALD, Steffen, 63517 Rodenbach (DE); VERSECK, Stefan, 63456 Hanau (DE); DEITING, Uta, 63477 Maintal (DE); WECKBECKER, Christoph, 63584 Gründau-Lieblos (DE); HUTHMACHER, Klaus, 63571 Gelnhausen (DE); BINDER, Michael, 63594 Hasselroth-Niedermittlau (DE); KULA, Maria-Regina, 81477 München (DE); ODENDAHL, Konrad, 50937 Köln (DE)
(86) Internationale Anmeldenummer: PCT/EP2005/002134
(87) Internationale Veröffentlichungsnummer: WO 2005/093080

(56) Entgegenhaltungen:
- WO-A-02/070717
- JP-A- 5 284 982
- BRADY D ET AL: "Characterisation of nitrilase and nitrile hydratase biocatalytic systems." APPLIED MICROBIOLOGY AND BIOTECHNOLOGY. MAR 2004, Bd. 64, Nr. 1, März 2004 (2004-03), Seiten 76-85, XP002333496 ISSN: 0175-7598 & INGVORSEN K ET AL: "Microbial hydrolysis of organic nitriles and amides." CIBA FOUNDATION SYMPOSIUM. 1988, Bd. 140, 1988, Seiten 16-31, XP000881167 ISSN: 0300-5208
- KOMEDA H ET AL: "A novel transporter involved in cobalt uptake." PROCEEDINGS OF THE NATIONAL ACADEMY OF SCIENCES OF THE UNITED STATES OF AMERICA. 7 JAN 1997, Bd. 94, Nr. 1, 7. Januar 1997 (1997-01-07), Seiten 36-41, XP009050024 ISSN: 0027-8424 & KOBAYASHI M ET AL: "Cobalt proteins." EUROPEAN JOURNAL OF BIOCHEMISTRY / FEBS. APR 1999, Bd. 261, Nr. 1, April 1999 (1999-04), Seiten 1-9, ISSN: 0014-2956
- NOJIRI MASAKI ET AL: "Functional expression of nitrile hydratase in Escherichia coli: Requirement of a nitrile hydratase activator and post-translational modification of a ligand cysteine" JOURNAL OF BIOCHEMISTRY (TOKYO), Bd. 125, Nr. 4, April 1999 (1999-04), Seiten 696-704, XP009049760 ISSN: 0021-924X

### SEQUENCE LISTING

<110> Degussa AG
<120> Nitrilhydratase aus Rhodococcus opacus
<130> 040069 BT
<160> 25
<170> PatentIn version 3.1
<210> 1
   <211> 3146
   <212> DNA
   <213> Rhodococcus opacus
<220>
   <221> CDS
   <222> (1)..(708)
   <223>
<220>
   <221> CDS
   <222> (710)..(1327)
   <223>
<220>
   <221> CDS
   <222> (2076)..(3146)
   <223>
<400> 1
   atg aac ggc atc ttc gat cta ggc gga acc gac ggc atg ggg ccg gtc
   48
   Met Asn Gly Ile Phe Asp Leu Gly Gly Thr Asp Gly Met Gly Pro Val
   1               5                   10                  15
   gac aac gac aaa ggc acc gag ccg gtg ttc cgc tca gcg tgg gaa aag
   96
   Asp Asn Asp Lys Gly Thr Glu Pro Val Phe Arg Ser Ala Trp Glu Lys
               20                  25                  30
   gcc gcc ttc tcg atg ttc gca caa ggc gcc cga gct ggc ctc tac aac
   144
   Ala Ala Phe Ser Met Phe Ala Gln Gly Ala Arg Ala Gly Leu Tyr Asn
           35                  40                  45
   atc gac gag ttc cgg cac tgc gtc gag cag atg gac ccc gcc gag tat
   192
   Ile Asp Glu Phe Arg His Cys Val Glu Gln Met Asp Pro Ala Glu Tyr
       50                  55                  60
   tta cta tcg aac tac tac gag cac tgg acg cat gcc gtc gaa cac ttc
   240
   Leu Leu Ser Asn Tyr Tyr Glu His Trp Thr His Ala Val Glu His Phe
   65                  70                  75                  80
   gcc cag caa aag aac ctc atc aca gcg gca gag ctc gaa aag cgc acg
   288
   Ala Gln Gln Lys Asn Leu Ile Thr Ala Ala Glu Leu Glu Lys Arg Thr
                   85                  90                      95
   cat ttc tac cgg gat aac cca gaa gcc ccc ctt ccg gag cgc aag gac
   336
   His Phe Tyr Arg Asp Asn Pro Glu Ala Pro Leu Pro Glu Arg Lys Asp
               100                 105                     110
   cca gag ctc ctc gac ttc gtg aac acc gcg atc gcg aac ggt ttc gcg
   384
   Pro Glu Leu Leu Asp Phe Val Asn Thr Ala Ile Ala Asn Gly Phe Ala
           115                 120                     125
   gcc tcc cgt gaa acc aat agg tcg gca gca ttc acc atc ggc gac cag
   432
   Ala Ser Arg Glu Thr Asn Arg Ser Ala Ala Phe Thr Ile Gly Asp Gln
       130                 135                     140
   gta ctg att gct gcg gac agt cca ttc gga cac acc cga cgg gcc ggc
   480
   Val Leu Ile Ala Ala Asp Ser Pro Phe Gly His Thr Arg Arg Ala Gly
   145                 150                     155                 160
   tac atc cgc ggt aag acc gga gtc atc acc gcg aca cac ggc gcc tac
   528
   Tyr Ile Arg Gly Lys Thr Gly Val Ile Thr Ala Thr His Gly Ala Tyr
                   165                 170                     175
   gtc tat ccc gac acc gcc ggt aac ggg ctc ggt gag tgc cca gag cac
   576
   Val Tyr Pro Asp Thr Ala Gly Asn Gly Leu Gly Glu Cys Pro Glu His
               180                 185                     190
   gtc tac acc gtg aag ttc acc gcc acc gaa ctt tgg ggc gaa cag agc
   624
   Val Tyr Thr Val Lys Phe Thr Ala Thr Glu Leu Trp Gly Glu Gln Ser
           195                 200                     205
   ggt gat cgc cac agc acc gtc tat ttc gat gtc tgg gaa ccg tac ctc
   672
   Gly Asp Arg His Ser Thr Val Tyr Phe Asp Val Trp Glu Pro Tyr Leu
       210                 215                     220
   tcg ctc gct acc gca ccc tct act cga gga atc tga c atg gcc gaa cag
   721
   Ser Leu Ala Thr Ala Pro Ser Thr Arg Gly Ile        Met Ala Glu Gln
   225                 230                     235
   cgc acc gac acc caa ttg cgt aca cac gaa gaa gtc gtc gcc cga gtc
   769
   Arg Thr Asp Thr Gln Leu Arg Thr His Glu Glu Val Val Ala Arg Val
   240                 245                     250                 255
   aag gcg ctc gag gcg ctg ctg atc gag aaa ggc gtc atg acg acc gag
   817
   Lys Ala Leu Glu Ala Leu Leu Ile Glu Lys Gly Val Met Thr Thr Glu
                   260                 265                     270
   gcc gtc gac cgg atg gcc gag gta tac gag aac gaa gtc ggc ccc cag
   865
   Ala Val Asp Arg Met Ala Glu Val Tyr Glu Asn Glu Val Gly Pro Gln
               275                 280                 285
   atc ggc gct cag att gtc gcc aag gcg tgg acc gac ccg aag ttc aag
   913
   Ile Gly Ala Gln Ile Val Ala Lys Ala Trp Thr Asp Pro Lys Phe Lys
           290                 295                 300
   aag agg ttg ctg gcc aat gcc acg act gcc tgc gca gag atg ggc tac
   961
   Lys Arg Leu Leu Ala Asn Ala Thr Thr Ala Cys Ala Glu Met Gly Tyr
       305                 310                 315
   ggc ggt ctg cag ggc gaa gac atg atc gtc gtg gaa aac acc gac acc
   1009
   Gly Gly Leu Gln Gly Glu Asp Met Ile Val Val Glu Asn Thr Asp Thr
   320                 325                 330                 335
   gta cac aac gcg att gtg tgc acc ctc tgc tcc tgc tac ccg tgg ccg
   1057
   Val His Asn Ala Ile Val Cys Thr Leu Cys Ser Cys Tyr Pro Trp Pro
                   340                 345                 350
   gtc ttg ggc ctg cca ccg aac tgg tac aag gca ccg gct tac cgc gca
   1105
   Val Leu Gly Leu Pro Pro Asn Trp Tyr Lys Ala Pro Ala Tyr Arg Ala
               355                 360                 365
   cgg atc gtg cgc gaa ccg cgg aag gtc ctc gcc gag gac ttc gac ttt
   1153
   Arg Ile Val Arg Glu Pro Arg Lys Val Leu Ala Glu Asp Phe Asp Phe
           370                 375                 380
   ccc atc ccc gac gac gtc gag atc cgc gtg tgg gac tcg agc gcc gag
   1201
   Pro Ile Pro Asp Asp Val Glu Ile Arg Val Trp Asp Ser Ser Ala Glu
       385                 390                 395
   ctg cgc tat tgg gtt tta ccg cag cgc cct gca cac acc gaa aga ttg
   1249
   Leu Arg Tyr Trp Val Leu Pro Gln Arg Pro Ala His Thr Glu Arg Leu
   400                 405                 410                 415
   acg gaa tcc gag ctg gta gcg ctg gtc acc cgc gac tcg atg atc ggt
   1297
   Thr Glu Ser Glu Leu Val Ala Leu Val Thr Arg Asp Ser Met Ile Gly
                   420                 425                 430
   gtg gga ccg gtg agg gag gcg atg tcg tga gcacgcgcat tgacgcaacc
   1347
   Val Gly Pro Val Arg Glu Ala Met Ser
               435                 440
   gagctcgggg aagcacgccg gcgaatcgag gcgttggtgt gtgatctgcc cggtggtgac
   1407
   gtaggctcac gcgccttcaa cgagccgtgg gaattgcgtg ccttcgcgat ggccgttgcc
   1467
   gtgtatcacc agggtcacta cgaatggagt gagtttcagc tctccctgat cgcgtcgatc
   1527
   cgccactggg agcagggcga gggaagggag ccgtggagct actacgagca ctggctcaat
   1587
   gcgctcgagt cggtactcgc cgccagcggc gccttatcgg acgcagtggg cctcgatgag
   1647
   cgcacgcgcg aagttctcac caccccacgg aacacgaacc accaccatgc acatcgcgaa
   1707
   cccgtcgcga tctcatctgc ggtgaactaa cccgcggcgc tactcgtccg ctggccagct
   1767
   ctctgcctgc tgtccagcga acgacacctc cgtgacagct tctcgttcac cgacccgatc
   1827
   actgattccc gacgcggtta ccaacgagca cccgcgtata aacagaaccg caaaggtatc
   1887
   gcagctgtcg gggacgagcg aatagcggat cgctcgcggg ggccggaccc atgcagctga
   1947
   tgctgctttc gcccgaatag cccagatatc cactggacga ggtgcgaggc ccgatacaag
   2007
   gcgagcgtca gcaaccggca aaccacagcg tccagagcca gcaccgtcat gtctagaaga
   2067
   ggaaagca ttg acg att act acc act tcg cca agg cag atc gcc ggt cgg
   2117
   Leu Thr Ile Thr Thr Thr Ser Pro Arg Gln Ile Ala Gly Arg
   445 450
   tgg aca cgt gcc gag cgg caa cga ctg agc gct atc atc ggc acc atc
   2165
   Trp Thr Arg Ala Glu Arg Gln Arg Leu Ser Ala Ile Ile Gly Thr Ile
   455 460 465 470
   gca ttg ctg cac gtg cta ggt atc gca atg tat ctc ggg cgc tcg ggt
   2213
   Ala Leu Leu His Val Leu Gly Ile Ala Met Tyr Leu Gly Arg Ser Gly
   475 480 485
   aac ccg gcc gcc gct ggt agc ctg gct ggc tcg gga ctg ctc gcc tat
   2261
   Asn Pro Ala Ala Ala Gly Ser Leu Ala Gly Ser Gly Leu Leu Ala Tyr
   490 495 500
   gtc ctg ggt gcg cgg cac gcg ttc gat gcc gac cac atc gcg gcc atc
   2309
   Val Leu Gly Ala Arg His Ala Phe Asp Ala Asp His Ile Ala Ala Ile
   505 510 515
   gac gac acc acc cgc atc atg ctc ctt cgc gga cgc cga ccc gtc ggc
   2357
   Asp Asp Thr Thr Arg Ile Met Leu Leu Arg Gly Arg Arg Pro Val Gly
   520 525 530
   gtc gga ttc ttt ttc gcc atg ggg cat tcg act gtc gtc ctc gtt ctc
   2405
   Val Gly Phe Phe Phe Ala Met Gly His Ser Thr Val Val Leu Val Leu
   535 540 545 550
   tct ctg atc gtc gct ttc gga gcg ggc tcg ctc agt tcg atg gaa gcg
   2453
   Ser Leu Ile Val Ala Phe Gly Ala Gly Ser Leu Ser Ser Met Glu Ala
   555 560 565
   tcc cgg gtc gag gag atc gga ggt tac gtc gcg acc tgc gtg gca gtg
   2501
   Ser Arg Val Glu Glu Ile Gly Gly Tyr Val Ala Thr Cys Val Ala Val
   570 575 580
   ctg ttc ttg gtg ctg gtg gcc gca ctc aac agt ttc gtt ctg cgc aag
   2549
   Leu Phe Leu Val Leu Val Ala Ala Leu Asn Ser Phe Val Leu Arg Lys
   585 590 595
   ctc ctc gct ctg tct cgt cgg atg cgc act ggg gaa gat atc tcc ggc
   2597
   Leu Leu Ala Leu Ser Arg Arg Met Arg Thr Gly Glu Asp Ile Ser Gly
   600 605 610
   gac ctc gag cgc ggg ctg ggt gaa cgg gga ttg ctc agc tgg ctt ctc
   2645
   Asp Leu Glu Arg Gly Leu Gly Glu Arg Gly Leu Leu Ser Trp Leu Leu
   615 620 625 630
   agc ggc cga ttg cgc ggg ctg att cgt tcg tcc tgg cac atg tac ccg
   2693
   Ser Gly Arg Leu Arg Gly Leu Ile Arg Ser Ser Trp His Met Tyr Pro
   635 640 645
   gtg ggc ctg ctc atg ggt ctc ggc ctg gaa acc gca tcc gaa gtg aca
   2741
   Val Gly Leu Leu Met Gly Leu Gly Leu Glu Thr Ala Ser Glu Val Thr
   650 655 660
   ttg ctg tct ctc act gcc tcc gca gcg agc gga ggt cag cta tcg cta
   2789
   Leu Leu Ser Leu Thr Ala Ser Ala Ala Ser Gly Gly Gln Leu Ser Leu
   665 670 675
   atg gcg att gtg agc ctt cca ttg ttg ttt gcc gcg ggg atg agc acc
   2837
   Met Ala Ile Val Ser Leu Pro Leu Leu Phe Ala Ala Gly Met Ser Thr
   680 685 690
   ttc gat act gca gac tca ctc gtc atg acc cgc gcc tat tcg tgg tcc
   2885
   Phe Asp Thr Ala Asp Ser Leu Val Met Thr Arg Ala Tyr Ser Trp Ser
   695 700 705 710
   tat aac gat gcc cag cgc cgc ctt cgc ttc aac act gta acc acg ggt
   2933
   Tyr Asn Asp Ala Gln Arg Arg Leu Arg Phe Asn Thr Val Thr Thr Gly
                   715                     720                 725
   gcg acc atg gtc atc ggg ttc ttc gtc gcg gga atc tac gtt tct gga
   2981
   Ala Thr Met Val Ile Gly Phe Phe Val Ala Gly Ile Tyr Val Ser Gly
               730                 735                 740
   ctg ctt gcg ccg cta cca ggg ttc ggt tgg ctg acc cct ctg ggc gcg
   3029
   Leu Leu Ala Pro Leu Pro Gly Phe Gly Trp Leu Thr Pro Leu Gly Ala
           745                 750                 755
   atc gcc gac aac ttc gag ttc ctc ggc tac gca gtc gcc gga ttg ttc
   3077
   Ile Ala Asp Asn Phe Glu Phe Leu Gly Tyr Ala Val Ala Gly Leu Phe
       760                 765                 770
   gtt gct acc tgg gca atc gca gca ctg gtt agc cgg cct cga cgg ctt
   3125
   Val Ala Thr Trp Ala Ile Ala Ala Leu Val Ser Arg Pro Arg Arg Leu
   775                 780                 785                 790
   gtc ggc agc tcg aag gtg tga
   3146
   Val Gly Ser Ser Lys Val
                   795
<210> 2
   <211> 235
   <212> PRT
   <213> Rhodococcus opacus
<400> 2
   Met Asn Gly Ile Phe Asp Leu Gly Gly Thr Asp Gly Met Gly Pro Val
   1               5                   10                  15
   Asp Asn Asp Lys Gly Thr Glu Pro Val Phe Arg Ser Ala Trp Glu Lys
               20                  25                  30
   Ala Ala Phe Ser Met Phe Ala Gln Gly Ala Arg Ala Gly Leu Tyr Asn
           35                  40                  45
   Ile Asp Glu Phe Arg His Cys Val Glu Gln Met Asp Pro Ala Glu Tyr
       50                  55                  60
   Leu Leu Ser Asn Tyr Tyr Glu His Trp Thr His Ala Val Glu His Phe
   65                  70                  75                  80
   Ala Gln Gln Lys Asn Leu Ile Thr Ala Ala Glu Leu Glu Lys Arg Thr
   85 90 95
   His Phe Tyr Arg Asp Asn Pro Glu Ala Pro Leu Pro Glu Arg Lys Asp
   100 105 110
   Pro Glu Leu Leu Asp Phe Val Asn Thr Ala Ile Ala Asn Gly Phe Ala
   115 120 125
   Ala Ser Arg Glu Thr Asn Arg Ser Ala Ala Phe Thr Ile Gly Asp Gln
   130 135 140
   Val Leu Ile Ala Ala Asp Ser Pro Phe Gly His Thr Arg Arg Ala Gly
   145 150 155 160
   Tyr Ile Arg Gly Lys Thr Gly Val Ile Thr Ala Thr His Gly Ala Tyr
   165 170 175
   Val Tyr Pro Asp Thr Ala Gly Asn Gly Leu Gly Glu Cys Pro Glu His
   180 185 190
   Val Tyr Thr Val Lys Phe Thr Ala Thr Glu Leu Trp Gly Glu Gln Ser
   195 200 205
   Gly Asp Arg His Ser Thr Val Tyr Phe Asp Val Trp Glu Pro Tyr Leu
   210 215 220
   Ser Leu Ala Thr Ala Pro Ser Thr Arg Gly Ile
   225 230 235
<210> 3
   <211> 205
   <212> PRT
   <213> Rhodococcus opacus
<400> 3
   Met Ala Glu Gln Arg Thr Asp Thr Gln Leu Arg Thr His Glu Glu Val
   1 5 10 15
   Val Ala Arg Val Lys Ala Leu Glu Ala Leu Leu Ile Glu Lys Gly Val
   20 25 30
   Met Thr Thr Glu Ala Val Asp Arg Met Ala Glu Val Tyr Glu Asn Glu
   35 40 45
   Val Gly Pro Gln Ile Gly Ala Gln Ile Val Ala Lys Ala Trp Thr Asp
   50 55 60
   Pro Lys Phe Lys Lys Arg Leu Leu Ala Asn Ala Thr Thr Ala Cys Ala
   65 70 75 80
   Glu Met Gly Tyr Gly Gly Leu Gln Gly Glu Asp Met Ile Val Val Glu
   85 90 95
   Asn Thr Asp Thr Val His Asn Ala Ile Val Cys Thr Leu Cys Ser Cys
   100 105 110
   Tyr Pro Trp Pro Val Leu Gly Leu Pro Pro Asn Trp Tyr Lys Ala Pro
   115 120 125
   Ala Tyr Arg Ala Arg Ile Val Arg Glu Pro Arg Lys Val Leu Ala Glu
   130 135 140
   Asp Phe Asp Phe Pro Ile Pro Asp Asp Val Glu Ile Arg Val Trp Asp
   145 150 155 160
   Ser Ser Ala Glu Leu Arg Tyr Trp Val Leu Pro Gln Arg Pro Ala His
   165 170 175
   Thr Glu Arg Leu Thr Glu Ser Glu Leu Val Ala Leu Val Thr Arg Asp
   180 185 190
   Ser Met Ile Gly Val Gly Pro Val Arg Glu Ala Met Ser
   195 200 205
<210> 4
   <211> 356
   <212> PRT
   <213> Rhodococcus opacus
<400> 4
   Leu Thr Ile Thr Thr Thr Ser Pro Arg Gln Ile Ala Gly Arg Trp Thr
   1 5 10 15
   Arg Ala Glu Arg Gln Arg Leu Ser Ala Ile Ile Gly Thr Ile Ala Leu
   20 25 30
   Leu His Val Leu Gly Ile Ala Met Tyr Leu Gly Arg Ser Gly Asn Pro
   35 40 45
   Ala Ala Ala Gly Ser Leu Ala Gly Ser Gly Leu Leu Ala Tyr Val Leu
   50 55 60
   Gly Ala Arg His Ala Phe Asp Ala Asp His Ile Ala Ala Ile Asp Asp
   65 70 75 80
   Thr Thr Arg Ile Met Leu Leu Arg Gly Arg Arg Pro Val Gly Val Gly
   85 90 95
   Phe Phe Phe Ala Met Gly His Ser Thr Val Val Leu Val Leu Ser Leu
   100 105 110
   Ile Val Ala Phe Gly Ala Gly Ser Leu Ser Ser Met Glu Ala Ser Arg
   115 120 125
   Val Glu Glu Ile Gly Gly Tyr Val Ala Thr Cys Val Ala Val Leu Phe
   130 135 140
   Leu Val Leu Val Ala Ala Leu Asn Ser Phe Val Leu Arg Lys Leu Leu
   145 150 155 160
   Ala Leu Ser Arg Arg Met Arg Thr Gly Glu Asp Ile Ser Gly Asp Leu
   165 170 175
   Glu Arg Gly Leu Gly Glu Arg Gly Leu Leu Ser Trp Leu Leu Ser Gly
   180 185 190
   Arg Leu Arg Gly Leu Ile Arg Ser Ser Trp His Met Tyr Pro Val Gly
   195 200 205
   Leu Leu Met Gly Leu Gly Leu Glu Thr Ala Ser Glu Val Thr Leu Leu
   210 215 220
   Ser Leu Thr Ala Ser Ala Ala Ser Gly Gly Gln Leu Ser Leu Met Ala
   225 230 235 240
   Ile Val Ser Leu Pro Leu Leu Phe Ala Ala Gly Met Ser Thr Phe Asp
   245 250 255
   Thr Ala Asp Ser Leu Val Met Thr Arg Ala Tyr Ser Trp Ser Tyr Asn
   260 265 270
   Asp Ala Gln Arg Arg Leu Arg Phe Asn Thr Val Thr Thr Gly Ala Thr
   275 280 285
   Met Val Ile Gly Phe Phe Val Ala Gly Ile Tyr Val Ser Gly Leu Leu
   290 295 300
   Ala Pro Leu Pro Gly Phe Gly Trp Leu Thr Pro Leu Gly Ala Ile Ala
   305 310 315 320
   Asp Asn Phe Glu Phe Leu Gly Tyr Ala Val Ala Gly Leu Phe Val Ala
   325 330 335
   Thr Trp Ala Ile Ala Ala Leu Val Ser Arg Pro Arg Arg Leu Val Gly
   340 345 350
   Ser Ser Lys Val
   355
<210> 5
   <211> 3146
   <212> DNA
   <213> Rhodococcus opacus
<220>
   <221> CDS
   <222> (1324)..(1737)
   <223>
<400> 5
   atgaacggca tcttcgatct aggcggaacc gacggcatgg ggccggtcga caacgacaaa 60
   ggcaccgagc cggtgttccg ctcagcgtgg gaaaaggccg ccttctcgat gttcgcacaa 120
   ggcgcccgag ctggcctcta caacatcgac gagttccggc actgcgtcga gcagatggac 180
   cccgccgagt atttactatc gaactactac gagcactgga cgcatgccgt cgaacacttc 240
   gcccagcaaa agaacctcat cacagcggca gagctcgaaa agcgcacgca tttctaccgg 300
   gataacccag aagcccccct tccggagcgc aaggacccag agctcctcga cttcgtgaac 360
   accgcgatcg cgaacggttt cgcggcctcc cgtgaaacca ataggtcggc agcattcacc 420
   atcggcgacc aggtactgat tgctgcggac agtccattcg gacacacccg acgggccggc 480
   tacatccgcg gtaagaccgg agtcatcacc gcgacacacg gcgcctacgt ctatcccgac 540
   accgccggta acgggctcgg tgagtgccca gagcacgtct acaccgtgaa gttcaccgcc 600
   accgaacttt ggggcgaaca gagcggtgat cgccacagca ccgtctattt cgatgtctgg 660
   gaaccgtacc tctcgctcgc taccgcaccc tctactcgag gaatctgaca tggccgaaca 720
   gcgcaccgac acccaattgc gtacacacga agaagtcgtc gcccgagtca aggcgctcga 780
   ggcgctgctg atcgagaaag gcgtcatgac gaccgaggcc gtcgaccgga tggccgaggt 840
   atacgagaac gaagtcggcc cccagatcgg cgctcagatt gtcgccaagg cgtggaccga 900
   cccgaagttc aagaagaggt tgctggccaa tgccacgact gcctgcgcag agatgggcta 960
   cggcggtctg cagggcgaag acatgatcgt cgtggaaaac accgacaccg tacacaacgc 1020
   gattgtgtgc accctctgct cctgctaccc gtggccggtc ttgggcctgc caccgaactg 1080
   gtacaaggca ccggcttacc gcgcacggat cgtgcgcgaa ccgcggaagg tcctcgccga 1140
   ggacttcgac tttcccatcc ccgacgacgt cgagatccgc gtgtgggact cgagcgccga 1200
   gctgcgctat tgggttttac cgcagcgccc tgcacacacc gaaagattga cggaatccga 1260
   gctggtagcg ctggtcaccc gcgactcgat gatcggtgtg ggaccggtga gggaggcgat 1320
   gtc gtg agc acg cgc att gac gca acc gag ctc ggg gaa gca cgc cgg 1368
   Val Ser Thr Arg Ile Asp Ala Thr Glu Leu Gly Glu Ala Arg Arg
   1 5 10 15
   cga atc gag gcg ttg gtg tgt gat ctg ccc ggt ggt gac gta ggc tca 1416
   Arg Ile Glu Ala Leu Val Cys Asp Leu Pro Gly Gly Asp Val Gly Ser
   20 25 30
   cgc gcc ttc aac gag ccg tgg gaa ttg cgt gcc ttc gcg atg gcc gtt 1464
   Arg Ala Phe Asn Glu Pro Trp Glu Leu Arg Ala Phe Ala Met Ala Val
   35 40 45
   gcc gtg tat cac cag ggt cac tac gaa tgg agt gag ttt cag ctc tcc 1512
   Ala Val Tyr His Gln Gly His Tyr Glu Trp Ser Glu Phe Gln Leu Ser
   50 55 60
   ctg atc gcg tcg atc cgc cac tgg gag cag ggc gag gga agg gag ccg 1560
   Leu Ile Ala Ser Ile Arg His Trp Glu Gln Gly Glu Gly Arg Glu Pro
   65 70 75
   tgg agc tac tac gag cac tgg ctc aat gcg ctc gag tcg gta ctc gcc 1608
   Trp Ser Tyr Tyr Glu His Trp Leu Asn Ala Leu Glu Ser Val Leu Ala
   80 85 90 95
   gcc agc ggc gcc tta tcg gac gca gtg ggc ctc gat gag cgc acg cgc 1656
   Ala Ser Gly Ala Leu Ser Asp Ala Val Gly Leu Asp Glu Arg Thr Arg
   100 105 110
   gaa gtt ctc acc acc cca cgg aac acg aac cac cac cat gca cat cgc 1704
   Glu Val Leu Thr Thr Pro Arg Asn Thr Asn His His His Ala His Arg
   115 120 125
   gaa ccc gtc gcg atc tca tct gcg gtg aac taa cccgcggcgc tactcgtccg 1757
   Glu Pro Val Ala Ile Ser Ser Ala Val Asn
   130 135
   ctggccagct ctctgcctgc tgtccagcga acgacacctc cgtgacagct tctcgttcac 1817
   cgacccgatc actgattccc gacgcggtta ccaacgagca cccgcgtata aacagaaccg 1877
   caaaggtatc gcagctgtcg gggacgagcg aatagcggat cgctcgcggg ggccggaccc 1937
   atgcagctga tgctgctttc gcccgaatag cccagatatc cactggacga ggtgcgaggc 1997
   ccgatacaag gcgagcgtca gcaaccggca aaccacagcg tccagagcca gcaccgtcat 2057
   gtctagaaga ggaaagcatt gacgattact accacttcgc caaggcagat cgccggtcgg 2117
   tggacacgtg ccgagcggca acgactgagc gctatcatcg gcaccatcgc attgctgcac 2177
   gtgctaggta tcgcaatgta tctcgggcgc tcgggtaacc cggccgccgc tggtagcctg 2237
   gctggctcgg gactgctcgc ctatgtcctg ggtgcgcggc acgcgttcga tgccgaccac 2297
   atcgcggcca tcgacgacac cacccgcatc atgctccttc gcggacgccg acccgtcggc 2357
   gtcggattct ttttcgccat ggggcattcg actgtcgtcc tcgttctctc tctgatcgtc 2417
   gctttcggag cgggctcgct cagttcgatg gaagcgtccc gggtcgagga gatcggaggt 2477
   tacgtcgcga cctgcgtggc agtgctgttc ttggtgctgg tggccgcact caacagtttc 2537
   gttctgcgca agctcctcgc tctgtctcgt cggatgcgca ctggggaaga tatctccggc 2597
   gacctcgagc gcgggctggg tgaacgggga ttgctcagct ggcttctcag cggccgattg 2657
   cgcgggctga ttcgttcgtc ctggcacatg tacccggtgg gcctgctcat gggtctcggc 2717
   ctggaaaccg catccgaagt gacattgctg tctctcactg cctccgcagc gagcggaggt 2777
   cagctatcgc taatggcgat tgtgagcctt ccattgttgt ttgccgcggg gatgagcacc 2837
   ttcgatactg cagactcact cgtcatgacc cgcgcctatt cgtggtccta taacgatgcc 2897
   cagcgccgcc ttcgcttcaa cactgtaacc acgggtgcga ccatggtcat cgggttcttc 2957
   gtcgcgggaa tctacgtttc tggactgctt gcgccgctac cagggttcgg ttggctgacc 3017
   cctctgggcg cgatcgccga caacttcgag ttcctcggct acgcagtcgc cggattgttc 3077
   gttgctacct gggcaatcgc agcactggtt agccggcctc gacggcttgt cggcagctcg 3137
   aaggtgtga 3146
<210> 6
   <211> 137
   <212> PRT
   <213> Rhodococcus opacus
<400> 6
   Val Ser Thr Arg Ile Asp Ala Thr Glu Leu Gly Glu Ala Arg Arg Arg
   1 5 10 15
   Ile Glu Ala Leu Val Cys Asp Leu Pro Gly Gly Asp Val Gly Ser Arg
   20 25 30
   Ala Phe Asn Glu Pro Trp Glu Leu Arg Ala Phe Ala Met Ala Val Ala
   35 40 45
   Val Tyr His Gln Gly His Tyr Glu Trp Ser Glu Phe Gln Leu Ser Leu
   50 55 60
   Ile Ala Ser Ile Arg His Trp Glu Gln Gly Glu Gly Arg Glu Pro Trp
   65 70 75 80
   Ser Tyr Tyr Glu His Trp Leu Asn Ala Leu Glu Ser Val Leu Ala Ala
   85 90 95
   Ser Gly Ala Leu Ser Asp Ala Val Gly Leu Asp Glu Arg Thr Arg Glu
   100 105 110
   Val Leu Thr Thr Pro Arg Asn Thr Asn His His His Ala His Arg Glu
   115 120 125
   Pro Val Ala Ile Ser Ser Ala Val Asn
   130 135
<210> 7
   <211> 17
   <212> DNA
   <213> Artificial
<220>
   <223> Primer
<400> 7
   atgaayggha tyttcga
   17
<210> 8
   <211> 17
   <212> DNA
   <213> Artificial
<220>
   <223> Primer
<400> 8
   atccagtgyy hgtagta
   17
<210> 9
   <211> 19
   <212> DNA
   <213> Artificial
<220>
   <223> Primer
<400> 9
   cgaagacatg atcgtcgtg
   19
<210> 10
   <211> 16
   <212> DNA
   <213> Artificial
<220>
   <223> Primer
<400> 10
   accggtccca caccga
   16
<210> 11
   <211> 16
   <212> DNA
   <213> Artificial
<220>
   <223> Primer
<400> 11
   tcgaggagat cggagg
   16
<210> 12
   <211> 18
   <212> DNA
   <213> Artificial
<220>
   <223> Primer
<400> 12
   gtatcgaagg tgctcatc
   18
<210> 13
   <211> 15
   <212> DNA
   <213> Artificial
<220>
   <223> Primer
<400> 13
   cgcgggctgg gtgaa
   15
<210> 14
   <211> 36
   <212> DNA
   <213> Artificial
<220>
   <223> Primer
<400> 14
   cggcggaatt caagaaggag acccgcatat gaacgg
   36
<210> 15
   <211> 36
   <212> DNA
   <213> Artificial
<220>
   <223> Primer
<400> 15
   ggtgcaagct tggatcctgt cagattcctc gagtag
   36
<210> 16
   <211> 33
   <212> DNA
   <213> Artificial
<220>
   <223> Primer
<400> 16
   gcgaaggatc ctgcatgcat cgaaattaat acg
   33
<210> 17
   <211> 33
   <212> DNA
   <213> Artificial
<220>
   <223> Primer
<400> 17
   catcaagctt ttcgccatgg ctatatctcc ttc
   33
<210> 18
   <211> 37
   <212> DNA
   <213> Artificial
<220>
   <223> primer
<400> 18
   ctgacaggat ccaagaagga gatatagcca tggccga
   37
<210> 19
   <211> 37
   <212> DNA
   <213> Artificial
<220>
   <223> Primer
<400> 19
   gttgcaagct tggtaccgct caagacatcg cctccct
   37
<210> 20
   <211> 36
   <212> DNA
   <213> Artificial
<220>
   <223> Primer
<400> 20
   gtgggtacca agaaggaggc gatcatatga gcacgc
   36
<210> 21
   <211> 32
   <212> DNA
   <213> Artificial
<220>
   <223> Primer
<400> 21
   gcggacgagt agcgaagctt gttagttcac cg
   32
<210> 22
   <211> 36
   <212> DNA
   <213> Artificial
<220>
   <223> Primer
<400> 22
   tcaaagcttg aaggagatat acatatgacg attact
   36
<210> 23
   <211> 33
   <212> DNA
   <213> Artificial
<220>
   <223> Primer
<400> 23
   gtcaagcttg gtaccgacat ctcacacctt cga
   33
<210> 24
   <211> 6307
   <212> DNA
   <213> E. coli, Rhodococcus opacus
<400> 24
   aattcttaag aaggagatat acatatgaac ggcatcttcg atctaggcgg aaccgacggc
   60
   atggggccgg tcgacaacga caaaggcacc gagccggtgt tccgctcagc gtgggaaaag
   120
   gccgccttct cgatgttcgc acaaggcgcc cgagctggcc tctacaacat cgacgagttc
   180
   cggcactgcg tcgagcagat ggaccccgcc gagtatttac tatcgaacta ctacgagcac
   240
   tggacgcatg ccgtcgaaca cttcgcccag caaaagaacc tcatcacagc ggcagagctc
   300
   gaaaagcgca cgcatttcta ccgggataac ccagaagccc cccttccgga gcgcaaggac
   360
   ccagagctcc tcgacttcgt gaacaccgcg atcgcgaacg gtttcgcggc ctcccgtgaa
   420
   accaataggt cggcagcatt caccatcggc gaccaggtac tgattgctgc ggacagtcca
   480
   ttcggacaca cccgacgggc cggctacatc cgcggtaaga ccggagtcat caccgcgaca
   540
   cacggcgcct acgtctatcc cgacaccgcc ggtaacgggc tcggtgagtg cccagagcac
   600
   gtctacaccg tgaagttcac cgccaccgaa ctttggggcg aacagagcgg tgatcgccac
   660
   agcaccgtct atttcgatgt ctgggaaccg tacctctcgc tcgctaccgc accctctact
   720
   cgaggaatct gacaggatcc tgcatgcatc gaaattaata cgacgaaatt aatacgactc
   780
   actatagggc aattgcgatc accacaattc agcaaattgt gaacatcatc acgttcatct
   840
   ttccctggtt gccaatggcc cattttcctg tcagtaacga gaaggtcgcg aattcaggcg 900
   ctttttagac tggtcgtaat gaacaattct taagaaggag atatagccat ggccgaacag 960
   cgcaccgaca cccaattgcg tacacacgaa gaagtcgtcg cccgagtcaa ggcgctcgag 1020
   gcgctgctga tcgagaaagg cgtcatgacg accgaggccg tcgaccggat ggccgaggta 1080
   tacgagaacg aagtcggccc ccagatcggc gctcagattg tcgccaaggc gtggaccgac 1140
   ccgaagttca agaagaggtt gctggccaat gccacgactg cctgcgcaga gatgggctac 1200
   ggcggtctgc agggcgaaga catgatcgtc gtggaaaaca ccgacaccgt acacaacgcg 1260
   attgtgtgca ccctctgctc ctgctacccg tggccggtct tgggcctgcc accgaactgg 1320
   tacaaggcac cggcttaccg cgcacggatc gtgcgcgaac cgcggaaggt cctcgccgag 1380
   gacttcgact ttcccatccc cgacgacgtc gagatccgcg tgtgggactc gagcgccgag 1440
   ctgcgctatt gggttttacc gcagcgccct gcacacaccg aaagattgac ggaatccgag 1500
   ctggtagcgc tggtcacccg cgactcgatg atcggtgtgg gaccggtgag ggaggcgatg 1560
   tcttgagcgg taccaagaag gaggcgatca tatgagcacg cgcattgacg caaccgagct 1620
   cggggaagca cgccggcgaa tcgaggcgtt ggtgtgtgat ctgcccggtg gtgacgtagg 1680
   ctcacgcgcc ttcaacgagc cgtgggaatt gcgtgccttc gcgatggccg ttgccgtgta 1740
   tcaccagggt cactacgaat ggagtgagtt tcagctctcc ctgatcgcgt cgatccgcca 1800
   ctgggagcag ggcgagggaa gggagccgtg gagctactac gagcactggc tcaatgcgct 1860
   cgagtcggta ctcgccgcca gcggcgcctt atcggacgca gtgggcctcg atgagcgcac 1920
   gcgcgaagtt ctcaccaccc cacggaacac gaaccaccac catgcacatc gcgaacccgt 1980
   cgcgatctca tctgcggtga actaacaagc ttggctgttt tggcggatga gagaagattt 2040
   tcagcctgat acagattaaa tcagaacgca gaagcggtct gataaaacag aatttgcctg 2100
   gcggcagtag cgcggtggtc ccacctgacc ccatgccgaa ctcagaagtg aaacgccgta 2160
   gcgccgatgg tagtgtgggg tctccccatg cgagagtagg gaactgccag gcatcaaata 2220
   aaacgaaagg ctcagtcgaa agactgggcc tttcgtttta tctgttgttt gtcggtgaac 2280
   gctctcctga gtaggacaaa tccgccggga gcggatttga acgttgcgaa gcaacggccc 2340
   ggagggtggc gggcaggacg cccgccataa actgccaggc atcaaattaa gcagaaggcc 2400
   atcctgacgg atggcctttt tgcgtttcta caaactcttt tgtttatttt tctaaataca 2460
   ttcaaatatg tatccgctca tgagacaata accctgataa atgcttcaat aatattgaaa 2520
   aaggaagagt atgagtattc aacatttccg tgtcgccctt attccctttt ttgcggcatt 2580
   ttgccttcct gtttttgctc acccagaaac gctggtgaaa gtaaaagatg ctgaagatca 2640
   gttgggtgca cgagtgggtt acatcgaact ggatctcaac agcggtaaga tccttgagag 2700
   ttttcgcccc gaagaacgtt ttccaatgat gagcactttt aaagttctgc tatgtggcgc 2760
   ggtattatcc cgtgttgacg ccgggcaaga gcaactcggt cgccgcatac actattctca 2820
   gaatgacttg gttgagtact caccagtcac agaaaagcat cttacggatg gcatgacagt 2880
   aagagaatta tgcagtgctg ccataaccat gagtgataac actgcggcca acttacttct 2940
   gacaacgatc ggaggaccga aggagctaac cgcttttttg cacaacatgg gggatcatgt 3000
   aactcgcctt gatcgttggg aaccggagct gaatgaagcc ataccaaacg acgagcgtga 3060
   caccacgatg cctgtagcaa tggcaacaac gttgcgcaaa ctattaactg gcgaactact 3120
   tactctagct tcccggcaac aattaataga ctggatggag gcggataaag ttgcaggacc 3180
   acttctgcgc tcggcccttc cggctggctg gtttattgct gataaatctg gagccggtga
   3240
   gcgtgggtct cgcggtatca ttgcagcact ggggccagat ggtaagccct cccgtatcgt
   3300
   agttatctac acgacgggga gtcaggcaac tatggatgaa cgaaatagac agatcgctga
   3360
   gataggtgcc tcactgatta agcattggta actgtcagac caagtttact catatatact
   3420
   ttagattgat ttaaaacttc atttttaatt taaaaggatc taggtgaaga tcctttttga
   3480
   taatctcatg accaaaatcc cttaacgtga gttttcgttc cactgagcgt cagaccccgt
   3540
   agaaaagatc aaaggatctt cttgagatcc tttttttctg cgcgtaatct gctgcttgca
   3600
   aacaaaaaaa ccaccgctac cagcggtggt ttgtttgccg gatcaagagc taccaactct
   3660
   ttttccgaag gtaactggct tcagcagagc gcagatacca aatactgtcc ttctagtgta
   3720
   gccgtagtta ggccaccact tcaagaactc tgtagcaccg cctacatacc tcgctctgct
   3780
   aatcctgtta ccagtggctg ctgccagtgg cgataagtcg tgtcttaccg ggttggactc
   3840
   aagacgatag ttaccggata aggcgcagcg gtcgggctga acggggggtt cgtgcacaca
   3900
   gcccagcttg gagcgaacga cctacaccga actgagatac ctacagcgtg agctatgaga
   3960
   aagcgccacg cttcccgaag ggagaaaggc ggacaggtat ccggtaagcg gcagggtcgg
   4020
   aacaggagag cgcacgaggg agcttccagg gggaaacgcc tggtatcttt atagtcctgt
   4080
   cgggtttcgc cacctctgac ttgagcgtcg atttttgtga tgctcgtcag gggggcggag
   4140
   cctatggaaa aacgccagca acgcggcctt tttacggttc ctggcctttt gctggccttt
   4200
   tgctcacatg ttctttcctg cgttatcccc tgattctgtg gataaccgta ttaccgcctt
   4260
   tgagtgagct gataccgctc gccgcagccg aacgaccgag cgcagcgagt cagtgagcga
   4320
   ggaagcggaa gagcgcctga tgcggtattt tctccttacg catctgtgcg gtatttcaca
   4380
   ccgcatatat ggtgcactct cagtacaatc tgctctgatg ccgcatagtt aagccagtat
   4440
   acactccgct atcgctacgt gactgggtca tggctgcgcc ccgacacccg ccaacacccg
   4500
   ctgacgcgcc ctgacgggct tgtctgctcc cggcatccgc ttacagacaa gctgtgaccg
   4560
   tctccgggag ctgcatgtgt cagaggtttt caccgtcatc accgaaacgc gcgaggcagc
   4620
   tgcggtaaag ctcatcagcg tggtcgtgaa gcgattcaca gatgtctgcc tgttcatccg
   4680
   cgtccagctc gttgagtttc tccagaagcg ttaatgtctg gcttctgata aagcgggcca
   4740
   tgttaagggc ggttttttcc tgtttggtca cttgatgcct ccgtgtaagg gggaatttct
   4800
   gttcatgggg gtaatgatac cgatgaaacg agagaggatg ctcacgatac gggttactga
   4860
   tgatgaacat gcccggttac tggaacgttg tgagggtaaa caactggcgg tatggatgcg
   4920
   gcgggaccag agaaaaatca ctcagggtca atgccagcgc ttcgttaata cagatgtagg
   4980
   tgttccacag ggtagccagc agcatcctgc gatgcagatc cggaacataa tggtgcaggg
   5040
   cgctgacttc cgcgtttcca gactttacga aacacggaaa ccgaagacca ttcatgttgt
   5100
   tgctcaggtc gcagacgttt tgcagcagca gtcgcttcac gttcgctcgc gtatcggtga
   5160
   ttcattctgc taaccagtaa ggcaaccccg ccagcctagc cgggtcctca acgacaggag
   5220
   cacgatcatg cgcacccgtg gccaggaccc aacgctgccc gagatgcgcc gcgtgcggct
   5280
   gctggagatg gcggacgcga tggatatgtt ctgccaaggg ttggtttgcg cattcacagt
   5340
   tctccgcaag aattgattgg ctccaattct tggagtggtg aatccgttag cgaggtgccg
   5400
   ccggcttcca ttcaggtcga ggtggcccgg ctccatgcac cgcgacgcaa cgcggggagg
   5460
   cagacaaggt atagggcggc gcctacaatc catgccaacc cgttccatgt gctcgccgag
   5520
   gcggcataaa tcgccgtgac gatcagcggt ccagtgatcg aagttaggct ggtaagagcc 5580
   gcgagcgatc cttgaagctg tccctgatgg tcgtcatcta cctgcctgga cagcatggcc 5640
   tgcaacgcgg gcatcccgat gccgccggaa gcgagaagaa tcataatggg gaaggccatc 5700
   cagcctcgcg tcgcgaacgc cagcaagacg tagcccagcg cgtcggccgc catgccggcg 5760
   ataatggcct gcttctcgcc gaaacgtttg gtggcgggac cagtgacgaa ggcttgagcg 5820
   agggcgtgca agattccgaa taccgcaagc gacaggccga tcatcgtcgc gctccagcga 5880
   aagcggtcct cgccgaaaat gacccagagc gctgccggca cctgtcctac gagttgcatg 5940
   ataaagaaga cagtcataag tgcggcgacg atagtcatgc cccgcgccca ccggaaggag 6000
   ctgactgggt tgaaggctct caagggcatc ggtcgacgct ctcccttatg cgactcctgc 6060
   attaggaagc agcccagtag taggttgagg ccgttgagca ccgccgccgc aaggaatggt 6120
   gcatgcatgc atcgaaatta atacgacgaa attaatacga ctcactatag ggcaattgcg 6180
   atcaccacaa ttcagcaaat tgtgaacatc atcacgttca tctttccctg gttgccaatg 6240
   gcccattttc ctgtcagtaa cgagaaggtc gcgaattcag gcgcttttta gactggtcgt 6300
   aatgaac 6307
<210> 25
   <211> 6191
   <212> DNA
   <213> E. coli, Rhodococcus opacus
<400> 25
   aattcttaag aaggagatat acatatgacg attactacca cttcgccaag gcagatcgcc 60
   ggtcggtgga cacgtgccga gcggcaacga ctgagcgcta tcatcggcac catcgcattg 120
   ctgcacgtgc taggtatcgc aatgtatctc gggcgctcgg gtaacccggc cgccgctggt 180
   agcctggctg gctcgggact gctcgcctat gtcctgggtg cgcggcacgc gttcgatgcc 240
   gaccacatcg cggccatcga cgacaccacc cgcatcatgc tccttcgcgg acgccgaccc 300
   gtcggcgtcg gattcttttt cgccatgggg cattcgactg tcgtcctcgt tctctctctg 360
   atcgtcgctt tcggagcggg ctcgctcagt tcgatggaag cgtcccgggt cgaggagatc 420
   ggaggttacg tcgcgacctg cgtggcagtg ctgttcttgg tgctggtggc cgcactcaac 480
   agtttcgttc tgcgcaagct cctcgctctg tctcgtcgga tgcgcactgg ggaagatatc 540
   tccggcgacc tcgagcgcgg gctgggtgaa cggggattgc tcagctggct tctcagcggc 600
   cgattgcgcg ggctgattcg ttcgtcctgg cacatgtacc cggtgggcct gctcatgggt 660
   ctcggcctgg aaaccgcatc cgaagtgaca ttgctgtctc tcactgcctc cgcagcgagc 720
   ggaggtcagc tatcgctaat ggcgattgtg agccttccat tgttgtttgc cgcggggatg 780
   agcaccttcg atactgcaga ctcactcgtc atgacccgcg cctattcgtg gtcctataac 840
   gatgcccagc gccgccttcg cttcaacact gtaaccacgg gtgcgaccat ggtcatcggg 900
   ttcttcgtcg cgggaatcta cgtttctgga ctgcttgcgc cgctaccagg gttcggttgg 960
   ctgacccctc tgggcgcgat cgccgacaac ttcgagttcc tcggctacgc agtcgccgga 1020
   ttgttcgttg ctacctgggc aatcgcagca ctggttagcc ggcctcgacg gcttgtcggc 1080
   agctcgaagg tgtgagatgt cggtaccaag cttggctgtt ttggcggatg agagaagatt 1140
   ttcagcctga tacagattaa atcagaacgc agaagcggtc tgataaaaca gaatttgcct 1200
   ggcggcagta gcgcggtggt cccacctgac cccatgccga actcagaagt gaaacgccgt 1260
   agcgccgatg gtagtgtggg gtctccccat gcgagagtag ggaactgcca ggcatcaaat 1320
   aaaacgaaag gctcagtcga aagactgggc ctttcgtttt atctgttgtt tgtcggtgaa 1380
   cgctctcctg agtaggacaa atccgccggg agcggatttg aacgttgcga agcaacggcc 1440
   cggagggtgg cgggcaggac gcccgccata aactgccagg catcaaatta agcagaaggc 1500
   catcctgacg gatggccttt ttgcgtttct acaaactctt ttgtttattt ttctaaatac 1560
   attcaaatat gtatccgctc atgagacaat aaccctgata aatgcttcaa taatatcgtc 1620
   cattccgaca gcatcgccag tcactatggc gtgctgctag cgctatatgc gttgatgcaa 1680
   tttctatgcg cacccgttct cggagcactg tccgaccgct ttggccgccg cccagtcctg 1740
   ctcgcttcgc tacttggagc cactatcgac tacgcgatca tggcgaccac acccgtcctg 1800
   tggatcctct acgccggacg catcgtggcc ggcatcaccg gcgccacagg tgcggttgct 1860
   ggcgcctata tcgccgacat caccgatggg gaagatcggg ctcgccactt cgggctcatg 1920
   agcgcttgtt tcggcgtggg tatggtggca ggccccgtgg ccgggggact gttgggcgcc 1980
   atctccttgc atgcaccatt ccttgcggcg gcggtgctca acggcctcaa cctactactg 2040
   ggctgcttcc taatgcagga gtcgcataag ggagagcgtc gaccgatgcc cttgagagcc 2100
   ttcaacccag tcagctcctt ccggtgggcg cggggcatga ctatcgtcgc cgcacttatg 2160
   actgtcttct ttatcatgca actcgtagga caggtgccgg cagcgctctg ggtcattttc 2220
   ggcgaggacc gctttcgctg gagcgcgacg atgatcggcc tgtcgcttgc ggtattcgga 2280
   atcttgcacg ccctcgctca agccttcgtc actggtcccg ccaccaaacg tttcggcgag 2340
   aagcaggcca ttatcgccgg catggcggcc gacgcgctgg gctacgtctt gctggcgttc 2400
   gcgacgcgag gctggatggc cttccccatt atgattcttc tcgcttccgg cggcatcggg 2460
   atgcccgcgt tgcaggccat gctgtccagg caggtagatg acgaccatca gggacagctt 2520
   caaggatcgc tcgcggctct taccagccta acttcgatca ctggaccgct gatcgtcacg 2580
   gcgatttatg ccgcctcggc gagcacatgg aacgggttgg catggattgt aggcgccgcc 2640
   ctataccttg tctgcctccc cgcgttgcgt cgcggtgcat ggagccgggc cacctcgacc 2700
   tgaatggaag ccggcggcac ctcgctaacg gattcaccac tccaagaatt ggagccaatc 2760
   aattcttgcg gagaactgtg aatgcgcaaa ccaacccttg gcagaacata tccatcgcgt 2820
   ccgccatctc cagcagccgc acgcggcgca tctcgggcag cgttgggtcc tggccacggg 2880
   tgcgcatgat cgtgctcctg tcgttgagga cccggctagg ctggcggggt tgccttactg 2940
   gttagcagaa tgaatcaccg atacgcgagc gaacgtgaag cgactgctgc tgcaaaacgt 3000
   ctgcgacctg agcaacaaca tgaatggtct tcggtttccg tgtttcgtaa agtctggaaa 3060
   cgcggaagtc ccctacgtgc tgctgaagtt gcccgcaaca gagagtggaa ccaaccggtg 3120
   ataccacgat actatgactg agagtcaacg ccatgagcgg cctcatttct tattctgagt 3180
   tacaacagtc cgcaccgctg tccggtagct ccttccggtg ggcgcggggc atgactatcg 3240
   tcgccgcact tatgactgtc ttctttatca tgcaactcgt aggacaggtg ccggcagcgc 3300
   ccaacagtcc cccggccacg gggcctgcca ccatacccac gccgaaacaa gcgccctgca 3360
   ccattatgtt ccggatctgc atcgcaggat gctgctggct accctgtgga acacctacat 3420
   ctgtattaac gaagcgctaa ccgtttttat caggctctgg gaggcagaat aaatgatcat 3480
   atcgtcaatt attacctcca cggggagagc ctgagcaaac tggcctcagg catttgagaa 3540
   gcacacggtc acactgcttc cggtagtcaa taaaccggta aaccagcaat agacataagc 3600
   ggctatttaa cgaccctgcc ctgaaccgac gaccgggtcg aatttgcttt cgaatttctg 3660
   ccattcatcc gcttattatc acttattcag gcgtagcacc aggcgtttaa gggcaccaat 3720
   aactgcctta aaaaaattac gccccgccct gccactcatc gcagtactgt tgtaattcat 3780
   taagcattct gccgacatgg aagccatcac agacggcatg atgaacctga atcgccagcg 3840
   gcatcagcac cttgtcgcct tgcgtataat atttgcccat ggtgaaaacg ggggcgaaga 3900
   agttgtccat attggccacg tttaaatcaa aactggtgaa actcacccag ggattggctg 3960
   agacgaaaaa catattctca ataaaccctt tagggaaata ggccaggttt tcaccgtaac 4020
   acgccacatc ttgcgaatat atgtgtagaa actgccggaa atcgtcgtgg tattcactcc 4080
   agagcgatga aaacgtttca gtttgctcat ggaaaacggt gtaacaaggg tgaacactat 4140
   cccatatcac cagctcaccg tctttcattg ccatacgaat tccggatgag cattcatcag 4200
   gcgggcaaga atgtgaataa aggccggata aaacttgtgc ttatttttct ttacggtctt 4260
   taaaaaggcc gtaatatcca gctgaacggt ctggttatag gtacattgag caactgactg 4320
   aaatgcctca aaatgttctt tacgatgcca ttgggatata tcaacggtgg tatatccagt 4380
   gatttttttc tccattttag cttccttagc tcctgaaaat ctcgataact caaaaaatac 4440
   gcccggtagt gatcttattt cattatggtg aaagttggaa cctcttacgt gccgatcaac 4500
   gtctcatttt cgccaaaagt tggcccaggg cttcccggta tcaacaggga caccaggatt 4560
   tatttattct gcgaagtgat cttccgtcac aggtatttat tcggcgcaaa gtgcgtcggg 4620
   tgatgctgcc aacttactga tttagtgtat gatggtgttt ttgaggtgct ccagtggctt 4680
   ctgtttctat cagctgtccc tcctgttcag ctactgacgg ggtggtgcgt aacggcaaaa 4740
   gcaccgccgg acatcagcgc tagcggagtg tatactggct tactatgttg gcactgatga 4800
   gggtgtcagt gaagtgcttc atgtggcagg agaaaaaagg ctgcaccggt gcgtcagcag 4860
   aatatgtgat acaggatata ttccgcttcc tcgctcactg actcgctacg ctcggtcgtt
   4920
   cgactgcggc gagcggaaat ggcttacgaa cggggcggag atttcctgga agatgccagg
   4980
   aagatactta acagggaagt gagagggccg cggcaaagcc gtttttccat aggctccgcc
   5040
   cccctgacaa gcatcacgaa atctgacgct caaatcagtg gtggcgaaac ccgacaggac
   5100
   tataaagata ccaggcgttt cccctggcgg ctccctcgtg cgctctcctg ttcctgcctt
   5160
   tcggtttacc ggtgtcattc cgctgttatg gccgcgtttg tctcattcca cgcctgacac
   5220
   tcagttccgg gtaggcagtt cgctccaagc tggactgtat gcacgaaccc cccgttcagt
   5280
   ccgaccgctg cgccttatcc ggtaactatc gtcttgagtc caacccggaa agacatgcaa
   5340
   aagcaccact ggcagcagcc actggtaatt gatttagagg agttagtctt gaagtcatgc
   5400
   gccggttaag gctaaactga aaggacaagt tttggtgact gcgctcctcc aagccagtta
   5460
   cctcggttca aagagttggt agctcagaga accttcgaaa aaccgccctg caaggcggtt
   5520
   ttttcgtttt cagagcaaga gattacgcgc agaccaaaac gatctcaaga agatcatctt
   5580
   attaagcttg catgcctgca ggacggatcc ccgggtaccg agctcgaatt taatcagata
   5640
   aaatatttca agatttcagt gcaatttatc tcttcaaatg tagcacctga agtcagcccc
   5700
   atacgatata agttgtaatt ctcatgtttg acagcttatc atcgataagc tttaatgcgg
   5760
   tagtttatca cagttaaatt gctaacgcag tcaggcaccg tgtatgaaat ctaacaatgc
   5820
   gctcatcgtc atcctcggca ccgtcaccct ggatgctgta ggcataggct tggttatgcc
   5880
   ggtactgccg ggcctcttgc gggattagtc atgccccgcg cccaccggaa ggagctgact
   5940
   gggttgaagg ctctcaaggg catcggtcga cgctctccct tatgcgactc ctgcattagg
   6000
   aagcagccca gtagtaggtt gaggccgttg agcaccgccg ccgcaaggaa tggtgcatgc
   6060
   atcgatcacc acaattcagc aaattgtgaa catcatcacg ttcatctttc cctggttgcc
   6120
   aatggcccat tttcctgtca gtaacgagaa ggtcgcgaat tcaggcgctt tttagactgg
   6180
   tcgtaatgaa c
   6191

## Beschreibung

Die Erfindung betrifft einen Polynucleotidcluster aus Rhodococcus, der für Polypeptide mit der Aktivität einer Nitrilhydratase, eines dieses Enzym aktivierenden Hilfsproteins P15K und eines Kobalttransporters kodierende Nukleotidsequenzen enthält, damit transformierte Mikroorganismen, in denen die für diese Proteine kodierende Nukleotidsequenzen verstärkt vorliegen und die Verwendung der transformierten Mikroorganismen zur Herstellung von Amiden aus Nitrilen.

Nitrilhydratasen wurden bereits in großer Zahl in der Literatur beschrieben (Synthetic applications of nitrileconverting enzymes; Martinkova, Ludmila; Mylerova, Veronika; Current Organic Chemistry (2003), 7(13), 1279-1295). Für die Herstellung von Acrylamid im Maßstab von mehreren tausend Tonnen pro Jahr werden Nitrilhydratasen schon seit 1983 eingesetzt. Dieses biokatalytische Verfahren hat sich gegenüber den chemischen Verfahren als konkurrenzfähig erwiesen (Enzymic synthesis of acrylamide: a success story not yet over; Kobayashi, Michihiko; Nagasawa, Toru; Yamada, Trends in Biotechnology (1992), 10(11), 402-8).

Neben den Nitrilhydratasen, die für die Umsetzung von Acrylnitril eingesetzt werden können, wurden z.B. auch solche beschrieben, die besonders geeignet sind für die Umsetzung von Methacrylnitril (A nitrile hydratase of Pseudonocardia thermophila and the genes encoding and manufacture of the enzyme for conversion of nitriles to amides (EP 790310), 3-Cyanopyridin (Process for producing amides with Rhodococcus nitrile hydratase (WO 2002055670) oder 2-Hydroxynitrilen wie 2-Hydroxy-4-methylthiobutyronitril (A nitrile hydratase of Rhodococcus and its use in the manufacture of amides (WO 2002070717) and Enzymic conversion of α-hydroxynitriles to the corresponding α-hydroxyamides, acids or acid salts, (WO 9832872). Demgegenüber sind bisher keine Nitrilhydratasen bekannt, mit deren Hilfe 2-Aminonitrile effektiv umgesetzt werden können. Die Nitrilhydratase aus Rhodococcus sp. Cr4 setzt z. B. 2-Hydroxynitrile mit hoher Aktivität um, ein einfaches 2-Aminonitril wie Aminoacetonitril aber überhaupt nicht (WO 2002070717).

Die enzymatische Umsetzung von Aminonitrilen zu den entsprechenden Amiden eröffnet eine attraktive Syntheseroute zu Aminosäuren, da 2-Aminoamide auf einfache Weise verseift werden können (WO 2001060789). Dieser Prozess läuft unter milden Bedingungen, mit sehr hoher Selektivität und ohne die Bildung von Nebenprodukten wie Salzen ab, wie sie bei der chemischen Hydrolyse anfallen.

Alternativ können Amide auch mit Alkali- oder Erdalkalimetallhydroxiden zu den entsprechenden Salzen der Säuren umgesetzt werden. Besonders bevorzugt ist diese Vorgehensweise beim Einsatz von Calciumhydroxid für die Umsetzung von 4-Methylthio-α-hydroxybutyramid (MHA-Amid), da das Calciumsalz von MHA direkt als alternative Produktform zu Methionin oder MHA als Futtermittelzusatz eingesetzt werden kann.

Für die Herstellung eines Commodity-Produkts wie z.B. DL-Methionin reicht es jedoch nicht aus, einen Biokatalysator mit hoher Aktivität zur Verfügung zu stellen. Zur Erhöhung der Aktivität muß ein Expressionssystem für die zu verstärkenden Gene etabliert werden. Es bietet sich die heterologe Expression z.B. vor allem in Escherichia coli, Bacillus, Pseudomonas, Pichia, Sacharomyces oder Aspergillus an, da diese Mikroorganismen ein schnelles Wachstum besitzen, sehr hohe Zelldichten erreichen und molekularbiologische Tools verfügbar sind, die sehr hohe Expressionsniveaus erlauben (Lee SY (1996) High cell-density culture of Escherichia coli. TIBTECH 14:98-105; Riesenberg D, Guthke R (1999) High-cell-density cultivation of microorganisms. Appl Microbiol Biotechnol 51:422-430).

Es ist bekannt, dass für die heterologe Expression von Nitrilhydratasen mindestens 3 Gene co-exprimiert werden müssen. Neben den beiden Strukturgenen muß außerdem sowohl für eisenabhängige, als auch für Cobaltabhängige Enzyme ein entsprechendes Helferprotein verstärkt werden (Nojiri

M. et al., (1999) Functional expression of Nitrile hydratases in Escherichia coli: Requirement of a nitrile hydratase activator and a post-translational modification of a ligand cysteine. J Biochem 125: 696-704 und Overproduction of stereoselective nitrile hydratase from Pseudomonas putida 5B in Escherichia coli: activity requires a novel downstream protein, Wu, S.; Fallon, R. D.; Payne, M. S. Applied Microbiology and Biotechnology (1997), 48(6), 704-708).

Zusätzlich zu diesen 3 Genen wurde in Rhodococcus rhodochrous J1 in einem Gencluster neben den Strukturgenen und dem Gen des Helferproteins ein weiteres Gen gefunden, das für einen Cobalttransporter codiert (A novel transporter involved in cobalt uptake, Komeda, Hidenobu et al., Proceedings of the National Academy of Sciences of the United States of America (1997), 94(1), 36-41). Sowohl die Überexpression in Rhodococcus als auch die in E. coli führt zu einer erhöhten Aufnahme von Co²⁺-Ionen aus dem Kulturmedium. Ausserdem konnte gezeigt werden, daß bei Co-Expression des Cobalttransporters zusammen mit den 3 anderen Proteinen die gleiche Nitrilhydratase-Aktivität bei einer niedrigeren Co-Konzentration im Medium erreicht werden kann, verglichen mit der alleinigen Expression der Strukturgene und des Helferproteins. Allerdings tritt dieser Effekt in Rhodococcus gemäß Komeda et al. nur bei Konzentrationen unterhalb von 42 µM auf.

Aus der EP 0 362 829 ist die Fermentation von Rhodococcus rhodochrous in Gegenwart von Kobaltsalzen bekannt.

Aufgabe der Erfindung ist es, Nitrilhydratasen mit hoher Aktivität zugänglich zu machen, die insbesondere α-Aminonitrile zu Amiden umsetzen.

Die Erfindung beinhaltet folgende Gegenstände:
1. Isolierter Polynukleotidcluster aus Rhodococcus opacus, enthaltend vier Nukleotidsequenzen, die für vier Polypeptide mit Aminosäuresequenzen kodieren, die zu jeweils mindestens 90 % identisch sind mit dem in den Sequenzen SEQ ID NO:2 bis SEQ ID NO:4 und SEQ ID NO:6 enthaltenen Aminosäuresequenzen, wobei die Polypeptide die Aktivitäten einer Nitrilhydratase, bestehend aus α- und β-Untereinheit, des Hilfsproteins P15K und eines Kobalttransporters besitzen.
2. Polynukleotide gemäß 1., ausgewählt aus der Gruppe:
   a) Polynukleotid bestehend aus den Positionen 1 bis 708 der Nukleotidsequenz SEQ ID NO:1 oder der dazu komplementären Nukleotidsequenz,
   b) Polynukleotid mit einer Nukleotidsequenz, die der Sequenz aus a) im Bereich der Degeneriertheit des genetischen Codes entspricht,
   c) Polynukleotid, das mit den komplementären Sequenzen a) oder b) unter stringenten Bedingungen hybridisiert, wobei die stringenten Bedingungen das Waschen auf 0,1x SSC bei einer Temperatur von 50-68°C beinhalten und
   d) Polynukleotid mit einer Nukleotidsequenz aus a), b) oder c), die funktionsneutrale Sinnmutationen enthält,
   wobei die Polynukleotide für die β-Untereinheit der Nitrilhydratase kodieren.
3. Polynukleotide gemäß 1., ausgewählt aus der Gruppe:
   a) Polynukleotid bestehend aus den Positionen 710 bis 1327 der Nukleotidsequenz SEQ ID NO:1 oder der dazu komplementären Nukleotidsequenz,
   b) Polynukleotid mit einer Nukleotidsequenz, die der Sequenz aus a) im Bereich der Degeneriertheit des genetischen Codes entspricht,
   c) Polynukleotid, das mit den komplementären Sequenzen a) oder b) unter stringenten Bedingungen hybridisiert, wobei die stringenten Bedingungen das Waschen auf 0,1x SSC bei einer Temperatur von 50-68°C beinhalten und
   d) Polynukleotid mit einer Nukleotidsequenz aus a), b) oder c), die funktionsneutrale Sinnmutationen enthält,
   wobei die Polynukleotide für die α-Untereinheit der Nitrilhydratase kodieren.
4. Polypeptid gemäss 1. enthaltend die Aminosäuresequenzen, die zu mindestens 90 % identisch sind mit SEQ ID NO:2 und SEQ ID NO:3, wobei das Polypeptid die Aktivität einer Nitrilhydratase aufweist.
5. Sonde oder Primer, enthaltend mindestens 20 aufeinanderfolgende Nukleotide innerhalb der Positionen 1 bis 1327 aus der Nukleotidsequenz SEQ ID NO:1 oder seiner komplementären Form.
6. Isoliertes Polynukleotid gemäss 2), das unter stringenten Bedingungen mit dem Komplement mit den Positionen 1 bis 708 aus SEQ ID NO:1 hybridisiert, wobei die stringenten Bedingungen das Waschen in 0,1 x SSC bei einer Temperatur von 50 bis 68°C beinhalten.
7. Isoliertes Polynukleotid gemäss 4), das unter stringenten Bedingungen mit dem Komplement mit den Positionen 710 bis 1327 aus SEQ ID NO:1 hybridisiert, wobei die stringenten Bedingungen das Waschen in 0,1 x SSC bei einer Temperatur von 50 bis 68°C beinhalten.
8. Vektoren enthaltend (ein) Polynukleotid(e) ausgewählt aus 1) bis 3) und 6) bis 7).
9. Vektor pUD15, bestehend aus der Nukleotidsequenz SEQ ID No: 24.
10. Vektor pUD16, bestehend aus der Nukleotidsequenz SEQ ID NO:25.
11. Wirtszelle, transformiert oder transfektiert durch die Einführung eines Polynukleotids gemäß 1).
12. Wirtszelle, transformiert durch die Einführung eines Vektors gemäß 8) bis 10).
13. Transformierte Wirtszelle gemäß 11) oder 12) wobei die Wirtszelle ein Bakterium der Familie Enterobacteriaceae, insbesondere Escherichia ist.
14. Verfahren zur Herstellung von Amiden aus Nitrilen mit Nitrilhydratasen des Ursprungs Rhodococcus oder dieses Enzym enthaltenden Mikroorganismen, bei dem man
   a) einen transformierten Mikroorganismus, der überexprimierte Gene mit den Nukleotidsequenzen ausgewählt aus den Ansprüchen 1 bis 3 und 6, 7 enthält, in Gegenwart von 0,15 bis 4 mM Co²⁺, unter Bedingungen fermentiert, die zur Bildung der Nitrilhydratase führen,
   b) dieses Enzym im Mikroorganismus anreichern lässt, und
   c) dieses Enzym aus den Zellen isoliert, oder
   d) die Mikroorganismen erntet und als das Enzym enthaltende ruhende Zellen gewinnt, und
   e) das Enzym oder die enthaltenen Mikroorganismen mit Verbindungen der allgemeinen Formel 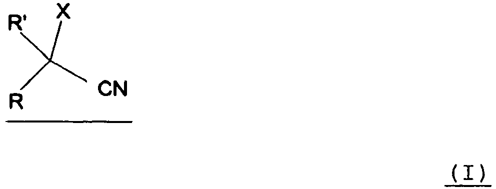 und

      R"-CN (II)

      in der bedeuten:
      X: OH, H, Alkyl mit 1 bis 4 C-Atomen, insbesondere NH2;
      R: H, gesättigter Alkylrest mit 1 bis 12 C-Atomen, verzweigt oder unverzweigt, gegebenenfalls NH2-substituiert, Alkenylreste mit 1 bis 12 C-Atomen, verzweigt
         oder
         unverzweigt, Cycloalkylgruppen mit 3 bis 6 C-Atomen,
         mit Alkylthiogruppen substituierte Alkylenreste,
         wobei Alkyl hier einem C₁ bis C₃-Rest
         und Alkylen einem zweiwertigen C₃ bis C₈-Rest
         entspricht,
      R': H, Alkyl mit 1 bis 3 C-Atomen,
      R": ein- oder zweikerniger ungesättigter Ring mit 6 bis 12 C-Atomen, gegebenenfalls mit einer oder zwei Alkylgruppen (C1 - C3), Cl, Br, F substituiert, einwertiger Alkylnitrilrest mit 1 bis 6 C-Atomen,
         zu den entsprechenden Amiden umsetzt.
15. Verfahren gemäss 14), dadurch gekennzeichnet, dass man Wirtszellen gemäss den Ansprüchen 11 bis 13 einsetzt.
16. Rekombinant gemäss 14) erzeugte Nitrilhydratase mit dem Ursprung Rhodococcus opacus, die α-Aminonitrile mit einer spezifischen Aktivität von > 50 U/mg Biotrockenmasse umsetzt.

Vektor DNA kann in eukaryonische oder prokaryonische Zellen durch bekannte Transformations- oder Transfektionstechniken eingeführt werden.

"Transformation", "Transfektion", "Konjugation" und "Transduktion" beziehen sich auf nach dem Stand der Techniken bekannten Maßnahmen, um fremde DNA einzuführen.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank von Rhodococcus opacus, die das vollständige Gen oder Teile davon enthält, mit einer Sonde, die die Sequenzen der erfindungsgemäßen Polynukleotide aus der SEQ ID No:1 oder Fragmente davon enthält und Isolierung der genannten Polynukleotidsequenz.

Polynukleotide, die die Sequenzen gemäß der Erfindung enthalten, sind als Hybridisierungs-Sonden für RNA, cDNA und DNA geeignet, um Nukleinsäuren beziehungsweise Polynukleotide oder Gene in voller Länge zu isolieren, die für die erfindungsgemäßen Proteine kodieren, oder um solche Nukleinsäuren beziehungsweise Polynukleotide oder Gene zu isolieren, die eine hohe Ähnlichkeit der Sequenzen mit denen der erfindungsgemäßen Gene aufweisen. Sie können ebenso als Sonde auf sogenannte "arrays", "micro arrays" oder "DNA chips" aufgebracht werden, um die entsprechenden Polynukleotide oder hiervon abgeleitete Sequenzen wie z.B. RNA oder cDNA zu detektieren und zu bestimmen.

Polynukleotide, die die Sequenzen gemäß der Erfindung enthalten, sind weiterhin als Primer geeignet, mit deren Hilfe mit der Polymerase-Kettenreaktion (PCR) DNA von Genen hergestellt werden kann, die für die erfindungsgemäßen Proteine kodieren.

Solche als Sonden oder Primer dienende Oligonukleotide, enthalten mindestens 25 oder 30, bevorzugt mindestens 20, ganz besonders bevorzugt mindestens 15 aufeinanderfolgende Nukleotide. Geeignet sind ebenfalls Oligonukleotide mit einer Länge von mindestens 40 oder 50 Nukleotiden. Gegebenenfalls sind auch Oligonukleotide mit einer Länge von mindestens 100, 150, 200, 250 oder 300 Nukleotiden geeignet.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Die Polynukleotide gemäß Erfindung schließen Polynukleotide gemäß SEQ ID No. 1 oder darin enthaltene Fragmente und auch solche ein, die zu wenigstens 90 %, 93 %, 95 %, 97 % oder 99% identisch sind mit den Polynukleotiden gemäß SEQ ID NO:1 oder darin enthaltenen Fragmenten.

Unter "Polypeptiden" versteht man Peptide oder Proteine, die zwei oder mehr über Peptidbindungen verbundene Aminosäuren enthalten.

Die Polypeptide gemäß Erfindung schließen ein Polypeptid gemäß den Sequenzen SEQ ID NO:2 bis SEQ ID NO:4 und SEQ ID NO:6, und auch solche ein, die zu wenigstens 90%, und besonders bevorzugt zu wenigstens 91%, 95%, 97% oder 99% identisch sind mit den Polypeptiden gemäß den Sequenzen SEQ ID NO:2 bis SEQ ID NO: 4 und SEQ ID NO:6.

Die Polynukleotide SEQ ID NO:1 enthält mehrere Einzelsequenzen, die für unterschiedliche Proteine kodieren. Die Sequenzen für die α-Untereinheit und das Helferprotein P15K überlappen sich.

Die Gene, die für die α- und β-Untereinheit der Nitrilhydratase kodieren, müssen gemeinsam exprimiert werden, um ein aktives Protein zu erhalten.

SEQ ID NO:2 gibt die Aminosäuresequenz der β-Untereinheit und SEQ ID NO:3 die der α-Untereinheit des Proteins wieder, das Nitrilhydrataseaktivität zeigt.

SEQ ID NO:2 leitet sich aus den Positionen 1 bis 708 und SEQ ID NO:3 aus den Positionen 710 bis 1327 der Nukleotidsequenz SEQ ID NO:1 ab.

Die Aminosäuresequenz des sogenannten Helferproteins P15K findet sich in SEQ ID NO:6, entsprechend den Positionen 1324 bis 1737 in der Nukleotidsequenz SEQ ID NO:1.

Das Helferprotein aktiviert die Nitrilhydratase und muss zusammen mit diesem Enzym in dem die Nitrilhydratase bildenden Mikroorganismus vorhanden sein.

SEQ ID NO:4 steht für die Aminosäuresequenz des Kobalttransporters und leitet sich aus den Positionen 2076 bis 3146 der Nukleotidsequenz SEQ ID NO:1 ab.

Das Startcodon ttg in SEQ ID NO:4 wird von PatentIN Version 3.1 mit Leucin, das Startcodon gtg in SEQ ID NO:6 mit Valin übersetzt. Es muss richtig heissen Methionin.

Es wurde gefunden, dass durch Co-Expression des Cobalttransporters die Nitrilhydrataseaktivität in E. coli um ein Vielfaches gesteigert wird. Dies gilt auch dann, wenn hohe Cobalt-Konzentrationen im Medium verwendet werden, die um Größenordnungen oberhalb der natürlich auftretenden Konzentrationen liegen. Überraschenderweise führt die co-Expression des Cobalttransporters nicht zu einer Vergiftung des Organismus sondern nur zu einer geringfügig gesteigerten Sensitivität der Zellen gegenüber hohen Cobaltkonzentrationen im Medium.

Zur Isolierung des erfindungsgemäßen Genclusters wird im allgemeinen zunächst eine Genbank dieses Mikroorganismus in Escherichia coli (E. coli) angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990), oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495-508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde.

Zur Herstellung einer Genbank in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E. coli Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige, für die Sequenzierung geeignete Vektoren subkloniert und anschließend sequenziert werden, so wie es z.B. bei Sanger et al. (Proceedings of the National Academy of Sciences of the United States of America, 74:5463-5467, 1977) beschrieben ist.

Die erhaltenen DNA-Sequenzen können dann mit bekannten Algorithmen bzw. Sequenzanalyse-Programmen wie z.B. dem von Staden (Nucleic Acids Research 14, 217-232(1986)), dem von Marck (Nucleic Acids Research 16, 1829-1836 (1988)) oder dem GCG-Programm von Butler (Methods of Biochemical Analysis 39, 74-97 (1998)) untersucht werden.

Kodierende DNA-Sequenzen, die sich aus den in SEQ ID No. 1 enthaltenen Sequenzen durch die Degeneriertheit des genetischen Kodes ergeben, sind ebenfalls Bestandteil der Erfindung. In gleicher Weise sind DNA-Sequenzen, die mit diesen Sequenzen oder Teilen von davon hybridisieren, Bestandteil der Erfindung. In der Fachwelt sind weiterhin konservative Aminosäureaustausche wie z.B. Austausch von Glycin gegen Alanin oder von Asparaginsäure gegen Glutaminsäure in Proteinen als "Sinnmutationen" ("sense mutations") bekannt, die zu keiner grundsätzlichen Veränderung der Aktivität des Proteins führen, d.h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie.

Schließlich sind DNA-Sequenzen Bestandteil der Erfindung, die durch die Polymerase-Kettenreaktion (PCR) unter Verwendung von Primern hergestellt werden, die sich aus SEQ ID NO: 1 ergeben. Derartige Oligonukleotide haben typischerweise eine Länge von mindestens 15 Nukleotiden.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 90% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird vorzugsweise bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C eingestellt wird. Es ist gegebenenfalls möglich die Salzkonzentration bis auf 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 90% bis 95% Identität zur Sequenz der eingesetzten Sonde besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Anleitungen zur Amplifikation von DNA-Sequenzen mit Hilfe der Polymerase-Kettenreaktion (PCR) findet der Fachmann unter anderem im Handbuch von Gait: Oligonukleotide synthesis: A Practical Approach (IRL Press, Oxford, UK, 1984) und bei Newton und Graham: PCR (Spektrum Akademischer Verlag, Heidelberg, Deutschland, 1994).

Im allgemeinen geht man so vor, dass man ein gut exprimierbares Gen in einen Vektor mit niedriger Kopienzahl, Gene mit schwächerer Expressionsleistung auf einem Vektor mit höherer Kopienzahl und/oder starkem Promotor kloniert. Die Wirtszellen sind mit diesen Vektoren in der Weise transformiert, dass sie im Vergleich zum Startorganismus mindestens jeweils eine zusätzliche Kopie der für die Bildung von Nitrilhydratase bzw. der weiteren Proteine kodierenden Nukleotidsequenzen enthalten.

Es hat sich als vorteilhaft erwiesen, das für den Kobalttransporter kodierende Gen in geringerem Umfang, beispielweise mit einem Vektor niedriger Kopienzahl - mindestens eine Kopie weniger - als die für die α- und β-Untereinheiten und das Helferprotein P15K kodierenden Polynukleotidsequenzen zu exprimieren. Die unterschiedliche Expression der genannten Gene kann auch durch die Verwendung unterschiedlich starker Promotoren erreicht werden.

Die für die α- und β-Untereinheit und das Helferprotein kodierenden Nukleotide liegen bevorzugt gemeinsam auf einem Vektor, mit einem gemeinsamen oder zwei getrennten Promotoren.

Die so hergestellten transformierten oder rekombinanten Mikroorganismen sind ebenfalls Teil der Erfindung.

Es wurde gefunden, dass die Verstärkung der für die Nitrilhydratase, das Helferprotein P15K und den Kobalttransporter kodierenden Gene in Mikroorganismen zu einer erhöhten Produktion der Nitrilhydratase oder auch zu einer erhöhten Aktivität der Nitrilhydratase führen.

Der Begriff "Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert.

Zur Erzielung einer Überexpression kann die Promotor- und Regluationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen Aminosäure-Produktion zu steigern. Durch Maßnahmen zur Verlängerung der Lebensdauer der m-RNA wird ebenfalls die Expression verbessert.

Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Durch die Maßnahmen der Verstärkung, insbesondere Überexpression, wird die Aktivität oder Konzentration des entsprechenden Proteins im allgemeinen um mindestens 10 %, 25 %, 50 %, 75 %, 100 %, 150 %, 200 %, 300 %, 400 % oder 500 %, maximal bis 1000 % oder 2000 %, bezogen auf die des Wildtyp-Proteins bzw. der Aktivität oder Konzentration des Proteins in nicht mit den erfindungsgemäßen Nukleotidsequenzen transformierten Mikroorganismen erhöht.

Ein anderer Gegenstand der Erfindung ist die Bereitstellung von in den ausgewählten Host-Stämmen im allgemeinen autonom replizierbaren Vektoren, die miteinander kompatibel sind und mindestens Nukleotidsequenzen gemäß den Ansprüche 2, 3 und 4 oder eine Nukleotidsequenz gemäß Anspruch 4 enthalten.

Vektor DNA kann in eukaryotische oder prokariotische Zellen durch bekannte Transformationstechniken eingeführt werden.

Als Host-Organismus dienen bevorzugt Mikroorganismen, für die es Expressionssysteme gibt, wie z. B. Pseudomonas, Pichia, verschiedene Hefen, Saccaromyces, Aspergillus oder der Familie Streptomyces, isbesondere E. coli. Mikroorganismen der Gattung Rhodococcus sind ebenso geeignet.

Gegenstand der Erfindung ist ebenso ein Verfahren zur Herstellung von Nitrilhydratase mit dem Ursprung Rhodococcus, insbesondere Rhodococcus opacus oder dieses Enzym enthaltende Mikroorganismen, bei dem man
a) einen transformierten Mikroorganismus, der überexprimierte Gene mit den Nukleotidsequenzen gemäß den Ansprüchen 1 bis 4 enthält, in Gegenwart von 0,15 bis 4 mM (mmol/1) Co²⁺, insbesondere 0,3 bis 4 mM, unter Bedingungen fermentiert, die zur Bildung der Nitrilhydratase führen,
b) dieses Enzym im Mikroorganismus anreichern lässt, und
c) dieses Enzym aus den Zellen isoliert, oder
d) die Mikroorganismen erntet und als das Enzym enthaltende ruhende Zellen gewinnt.

Die rekombinante erzeugte Nitrilhydratase setzt α-Aminonitrile mit einer Aktivität von > 50 U/mg Biotrockenmasse um.

Bevorzugt fermentiert man in der Gegenwart von 0,5 bis 3,5 mM Co²⁺, insbesondere 0,7 bis 3 mM das bevorzugt als lösliches Salz der Fermentationsbrühe zugesetzt wird.

Die erfindungsgemäß verwendeten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Lactose, Fructose, Maltose, Melasse, Stärke und Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Die genannten Einsatzstoffe können zur Kultur in Form eines einmaligen Ansatzes hinzugegeben oder in geeigneter weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe wie z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 10°C bis 40°c und vorzugsweise bei 10°C bis 30°C. Die Kultur wird bevorzugt mindestens solange fortgesetzt, bis sie die logarithmische Wachstumsphase durchschritten hat. Dieses Ziel wird normalerweise innerhalb von 10 Stunden bis 70 Stunden erreicht.

Gegenstand der Erfindung ist ebenfalls ein Verfahren zur enzymatischen Herstellung von Amiden aus Nitrilen, das folgende Schritte enthält:
a) Umsetzung einer Nitrilgruppen enthaltenden Verbindung mit einem Enzym aus Rhodococcus, insbesondere Rhodococcus opacus, das Nitrilhydratase-Aktivität aufweist, und
b) gegebenenfalls Abtrennung das Amids.

In einer Verfahrensvariante werden die Zellen geerntet, gewaschen und in einem Puffer als Suspension bei einem pH-Wert von 5 - 9, insbesondere von 6,8 bis 7,9 aufgenommen. Die Zellkonzentration der ruhenden Zellen beläuft sich auf im allgemeinen 1 - 25 %, insbesondere 1,5 bis 15 % (Feuchtgewicht/v). Sie können mit physikalischen oder chemischen Methoden so permeabilisiert werden, z. B. Toluol wie bei Wilms et al., J. Biotechnol., Vol 86 (2001), 19 - 30 beschrieben, dass die umzuwandelnden Nitrilverbindungen die Zellwand durchdringen und das entstandene Amid austreten kann.

Der Biokatalysator (Ganzzellkatalysator) zeigt eine ausgezeichnete Stabilität, so dass Produktkonzentrationen von über 100 g/l erreicht werden können.

Es ist auch möglich, die erfindungsgemäße Nitrilhydratase aus den Zellen nach bekannten Methoden abzutrennen, sie gegebenenfalls aufzureinigen und zur Umsetzung der Nitrile einzusetzen.

Gegenstand der Erfindung ist auch ein Verfahren, das dadurch gekennzeichnet ist, dass man Verbindungen der allgemeinen Formeln

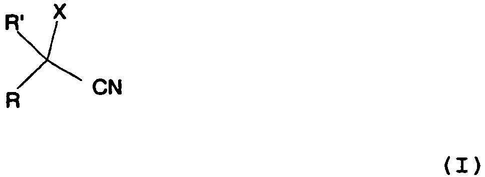

R"-CN (II)

in der bedeuten:
- X:: OH, H,Alkyl mit 1 bis 4 C-Atomen, Aryl, insbesondere NH₂;
- R:: H, gesättigter Alkylrest mit 1 bis 12 C-Atomen, verzweigt oder unverzweigt, gegebenenfalls mit NH2 substituiert, Alkenylreste mit 1 bis 12 C-Atomen, verzweigt oder unverzweigt, Cycloalkylgruppen mit 3 bis 6 C-Atomen, mit Alkylthiogruppen substituierte Alkylenreste, wobei Alkyl hier einem C₁ bis C₃-Rest und Alkylen einem zweiwertigen C₃ bis C₈-Rest entspricht,
- R':: H, Alkyl mit 1 bis 3 C-Atomen,
- R":: ein oder zweikerniger aromatischer Ring, mit 6 bis 12 C-Atomen, gegebenenfalls substituiert mit einer oder zwie Alkylgruppen (C1 - C3) oder Cl oder F, Alkylnitril mit 1 bis 6 C-Atomen.
zu den entsprechenden Amiden umsetzt.

Folgende Nitrile werden bevorzugt umsetzt:
gesättigte Mononitrile:
   Acetonitril, Propionitril, Butyronitril, Isobutyronitril, Valeronitril, Isovaleronitril, Capronitril
gesättigte Dinitrile:
   Malonitril, Succinonitril, Glutaronitril, Adiponitril
aromatische unsubstituierte und substituierte Mono- und Dinitrile:
   Benzonitril, 2,6-Difluorbenzonitril, Phthalonitril, Isophtalonitril, Terephthalonitril,
α-Aminonitrile:
   α-Aminopropionitril, α-Aminomethylthiobutyronitril, α-Aminobutyronitril, Aminoacetonitril, alle von natürlichen Aminosäuren abgeleiteten Nitrile, α-Amino-3,3-dimethylpropionitril α-Amino-2,3-dimethylpropionitril
Nitrile mit Carboxyl-Gruppen:
   Cyanessigsäure
β-Aminonitrile:
   3-Aminopropionitril
ungesättigte Nitrile:
   Acrylnitril, Methacrylonitril, Allylcyanid, Crotononitril
α-Hydroxynitrile:
   α-Hydroxy-n-propionitril, α-Hydroxy-n-butyronitril, α-Hydroxy-isobutyronitril, α-Hydroxy-n-hexanonitril, α-Hydroxy-n-heptanonitril, α-hydroxy-n-octyronitril, α, γ-Dihydroxy-β,β-dimethylbutyronitril, Acroleincyanohydrin, Methacrylaldehyd cyanohydrin, 3-Chlorolactonitril, 4-Methylthio-α-hydroxybutyronitril und α-Hydroxy-α-phenylpropionyl.

Die Konzentration der umzusetzenden Nitrile in der Reaktionslösung ist nicht auf bestimmte Bereiche begrenzt.

Um eine Inhibierung der Enzymaktivität durch das Substrat zu vermeiden, hält man die Konzentration des Nitrils im allgemeinen auf 0,001 bis 10 w/w%, insbesondere 0,1 bis 2 w/w%, bezogen auf die Menge des Biokatalysators als getrocknete Zellmasse. Das Substrat kann zu Beginn der Umsetzung insgesamt oder im Verlauf der Umsetzung kontinuierlich oder diskontinuierlich zugesetzt werden.

Wenn die Löslichkeit der Nitrilverbindung in dem wässrigen Reaktionssystem zu gering ist, kann ein Lösungsvermittler zugesetzt werden.

Die Reaktion kann aber alternativ auch in einem Zweiphasensystem Wasser/organische Lösungsmittel durchgeführt werden.

Bei der Verwendung von Zellen des Mikroorganismus als enzymatisch aktivem Material, verhält sich die Menge der eingesetzten Zellen zur Substratmenge bevorzugt auf 0,001 bis 8 w/w% als getrocknete Zellmasse.

Das Trockengewicht der Zellmasse wird mit einem Moisture Analyser MA45 (Sartorius) bestimmt.

Es ist auch möglich, das isolierte Enzym nach allgemein bekannten Techniken zu immobilisieren und in dieser Form dann einzusetzen.

Die Reaktion wird im allgemeinen bei Temperaturen von -5°C bis 50°C, insbesondere 0°C bis 30°C, und einer Zeitdauer von 0,1 bis 100 Stunden durchgeführt.

Der einzuhaltende pH-Wert des Reaktionsgemisches ist so lange nicht auf bestimmte Werte begrenzt, wie die enzymatische Aktivität nicht beeinträchtigt wird. Nach der Umsetzung kann das gebildete Amid aus der Reaktionslösung wie bekannt abgetrennt und gereinigt werden.

Gegenstand der Erfindung ist ebenfalls ein Verfahren, bei dem man das Amid bzw. die das Amid enthaltende Lösung z. B. von den Zellen der Biomasse abtrennt und das Amid entweder zu der entsprechenden Säure verseift oder unter Zusatz von Alkali- oder Erdalkalimetallhydroxiden zu den entsprechenden Salzen der Säuren umsetzt. Bevorzugt wird MHA-Amid it Calciumhydroxid verseift und das entsprechende Calciumsalz isoliert.

### Beispiele

### Beispiel 1

### Klonierung der Nitrilhydratase aus Rhodococcus opacus

Chromosomale DNA von Rhodococcus opacus wurde mit den Restriktionsenzymen PinAI, PstI und XmaI (Roche) verdaut und die Fragmente auf einem 0,8%igem Agarose-Gel aufgetrennt. Ein Southern Blot wurde nach Standard-Methoden (z. B. in Sambrook et al.: Molecular Cloning, A Laboratory Manual, Cold Spring Habor Laboratory Pess, 1989) auf eine positiv geladene Nylon-Membran (Hybond-N+, Amersham) durchgeführt. Die Hybridisierung erfolgte mit einer DIGmarkierten Sonde nach Anweisung des Herstellers (Roche). Die Sonde wurde mittels PCR mit den degenerierten Primern 1F und 1R mit genomischer DNA als Template hergestellt. Die Primer waren von homologen Regionen der β-Untereinheit abgeleitet, die durch Sequenz-Alignment verschiedener NHasen bestimmt wurden. Deren Sequenzen wurden aus Datenbanken erhalten. Zur Isolierung eines detektierten ca. 2,2 kb großen PinAI-Fragmentes wurden mit PinAI geschnittene DNA-Fragmente zwischen 2 und 2,5 kb über präparative Gelelektrophorese aufgereinigt, in den mit XmaI geschnittenen Vektor pUC18 (Promega) ligiert und der Ligationsansatz in E. coli JM109 (Promega) transformiert. Positive Transformanten wurden über Kolonie-Hybridisierung mit der gleichen Sonde identifiziert. Die so erhaltenen Klone enthielten ein Insert von 2206 nt mit dem Gen der β-Untereinheit und dem Großteil des Gens der α-Untereinheit der Nitrilhydratase.

Für die fehlende Sequenz wurde mit oben beschriebener Methode mit den Primern 2F und 2R eine neue Sonde hergestellt, die am 3'-Ende des klonierten PinAI-Fragmentes hybridisiert. Als Template diente das in pUC18 klonierte PinAI-Fragment. Vor der Hybridisierung mit dieser Sonde wurden von der oben beschriebenen Membran zunächst die Farbsignale sowie die erste Sonde nach Anweisung des Herstellers (Roche) entfernt. Auf dieser Membran wurde mit der zweiten Sonde eine ca. 2 kb große PstI-Bande detektiert. Wie oben beschrieben, wurde das entsprechende DNA-Fragment in den mit PstI geöffneten Vektor pUC18 kloniert, das Produkt in E. coli JM109 transformiert und positive Klone über Kolonie-Hybridisierung identifiziert. Das PstI-Fragment ist 1883 nt groß und enthält einen (3'-) Teil des Gens der α-Untereinheit der Nitrilhydratase, das Gen des Hilfsproteins P15K und einen (5'-) Teil des Gens des Kobalttransporters.

Um ein DNA-Fragment mit der fehlenden Sequenz des Kobalttransporter-Genes zu klonieren, wurde mit den Primern 3F und 3R und dem in pUC18 klonierten PstI-Fragment als Template eine neue Sonde hergestellt, die am 3'-Ende des klonierten PstI-Fragmentes hybridisiert. Mit dieser Sonde wurde auf der gleichen Membran, von der wiederum zuvor Farbsignale und die zweite Sonde entfernt worden waren, eine ca. 1,7 kb große XmaI-Bande detektiert. Das entsprechende DNA-Fragment wurde in den mit XmaI geöffneten Vektor pUC18 kloniert, das Produkt in E. coli JM109 transformiert und positive Klone über Kolonie-Hybridisierung identifiziert. Dazu wurde eine Sonde eingesetzt, die mit den Primern 4F und 3R amplifiziert worden war. Das XmaI-Fragment ist 1747 nt groß und enthält einen (3'-) Teil des Gens des Kobalttransporters.

Die fortlaufende Sequenz des Genclusters, der die Polynukleotide kodierend für die α-β-Untereinheiten der Nitrilhydratase, des Hilfsproteins P15K und des Kobalttransporters enthält, ist in SEQ ID NO:1 wiedergegeben.

### Beispiel 2

### Konstruktion der Expressionsvektoren

Die Strukturgene wurden in einen bekannten Expressionsvektor für E. coli kloniert, bei dem die eingefügten Gene unter der Kontrolle eines Rhamnose-Promoters stehen. Zusätzlich wurde ein zweiter Rhamnose-Promoter eingefügt. Das Gen für die β-UE wurde dazu mit den Primern 5F und 5R amplifiziert, die Schnittstellen für die Restriktionsenzyme NdeI, BamHI und HindIII einfügten. Der zweite Rhamnose-Promoter wurde mit den Primern 6F und 6R amplifiziert, die Schnittstellen für die Restriktionsenzyme BamHI, NcoI und HindIII einfügten. Das Gen für die α-UE wurde mit den Primern 7F und 7R amplifiziert, die Schnittstellen für die Restriktionsenzyme NcoI, KpnI und HindIII einfügten. Das Gen für das Protein P15K wurde mit den Primern 8F und 8R amplifiziert, die Schnittstellen für die Restriktionsenzyme KpnI und HindIIIeinfügten und das Startcodon von GTG zu ATG änderten. Der so entstandene Expressionsvektor heißt pUD 15.

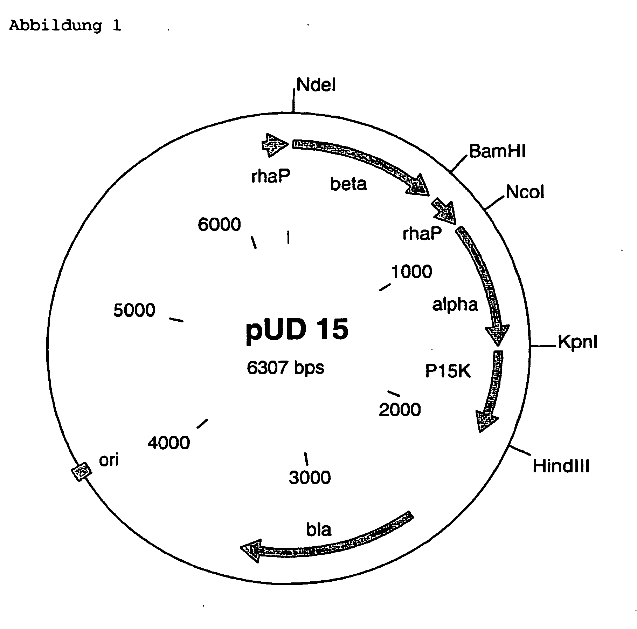

Die Restriktionskarte findet sich in Abbildung 1, die Sequenz unter SEQ ID NO:24.

Das Gen für den Kobalttransporter wurde in einen anderen Expressionsvektor für E. coli kloniert, bei dem die eingefügten Gene auch unter der Kontrolle des Rhamnose-Promoters stehen. Dazu wurde es mit den Primern 9F und 9R amplifiziert, die Schnittstellen für die Restriktionsenzyme NdeI und HindIII einfügten und das Startcodon von TTG zu ATG änderten. Der so entstandene Expressionsvektor heißt pUD 16.

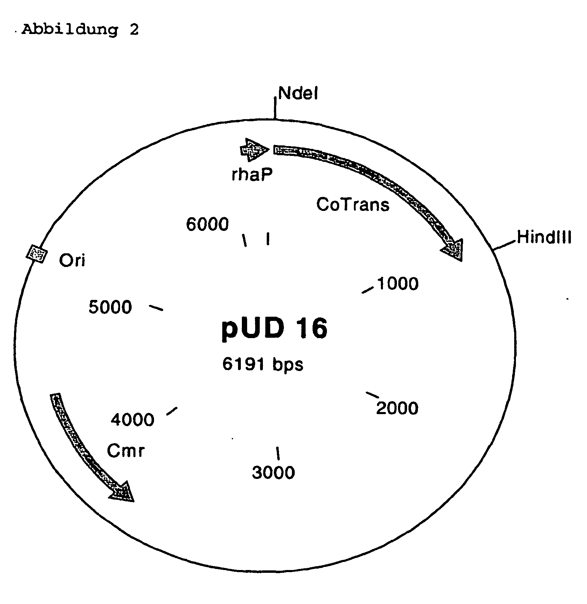

Die Restriktionskarte findet sich in Abbildung 2, die Sequenz unter SEQ ID NO:25.

Die Expressionsplasmide wurden in den Stamm E. coli DSM 14459 transformiert, der bei der Deutschen Sammlung von Mikroorganismen und Zellkulturen GmbH (DSMZ) hinterlegt ist.

### Primer:

| | |
|---|---|
| 1F | 5'-ATG AAY GGH ATY TTC GA-3' |
| 1R | 5'-ATC CAG TGY YHG TAG TA-3' |
| 2F | 5'-CGA AGA CAT GAT CGT CGT G-3' |
| 2R | 5'-ACC GGT CCC ACA CCG A-3' |
| 3F | 5'-TCG AGG AGA TCG GAG G-3' |
| 3R | 5'-GTA TCG AAG GTG CTC ATC-3' |
| 4F | 5'-CGC GGG CTG GGT GAA-3' |
| 5F | 5'-CGG CGG AAT TCA AGA AGG AGA CCC GCA TAT GAA CGG-3' |
| 5R | 5'-GGT GCA AGC TTGGAT CCT GTC AGA TTC CTC GAG TAG-3' |
| 6F | 5'-GCG AAG GAT CCT GCA TGC ATC GAA ATT AAT ACG-3' |
| 6R | 5'-CAT CAA GCT TTT CGC CAT GGC TAT ATC TCC TTC-3' |
| 7F | 5'-CTG ACA GGA TCC AAG AAG GAG ATA TAG CCA TGG CCG A-3' |
| 7R | 5'-GTT GCA AGC TTG GTA CCG CTC AAG ACA TCG CCT CCC T-3' |
| 8F | 5'-GTG GGT ACC AAG AAG GAG GCG ATC ATA TGA GCA CGC-3' |
| 8R | 5'-GCG GAC GAG TAG CGA AGC TTG TTA GTT CAC CG-3' |
| 9F | 5'-TCA AAG CTT GAA GGA GAT ATA CAT ATG ACG ATT ACT-3' |
| 9R | 5'-GTC AAG CTT GGT ACC GAC ATC TCA CAC CTT CGA-3' |

Die Gene befinden sich auf den Abschnitten:

| | | |
|---|---|---|
| pUD15: | Gen der β-Untereinheit: | nt 25 - 732 |
| | Gen der α-Untereinheit: | nt 949 - 1566 |
| | Gen von P15K: | nt 1592 - 2005 |
| | | |
| pUD16: | Gen des Kobalttransporters: | nt 25 - 1095 |

### Beispiel 3

### Heterologe Expression der Nitrilhydratase in E. coli DSM 14559

DSM 14559 wurde im Zusammenhang mit der DE 101 55 928 hinterlegt.

Die mit pUD15 transformierten Zellen wurden in LB-Medium (LB Bouillon nach Miller, VWR), das 1 mM CoCl₂ und 100 µg/ml Ampicillin enthielt unter Schütteln bei 37°C angezogen. Die Anzucht für die mit pUD15 und pUD16 transformierten Zellen wurde analog durchgeführt, das Medium enthielt aber zusätzlich 50 µg/ml Chloramphenicol. Die Zellen wurden danach noch 3 mal in das selbe Medium überimpft, nachdem sie mindestens eine OD₆₀₀ von 2 erreicht hatten. Nach 12 - 16 Stunden wurde eine solche Menge der letzten Vorkultur in eine Hauptkultur überimpft, dass diese eine OD₆₀₀ von 0,1 aufwies. Das Kulturmedium der Hauptkultur entsprach dem der Vorkultur, enthielt aber zusätzlich 2 g/L L-Rhamnose. Die Ernte der Zellen erfolgte nach 22 Stunden.

### Beispiel 4

### Bestimmung der enzymatischen Aktivität

Die Zellen wurden wie in Beispiel 3 beschrieben angezogen, durch Zentrifugation vom Kulturmedium abgetrennt und im Standardpuffer (50 mM Kaliumphosphatpuffer pH 7,5) resuspendiert. 50 µl dieser Zellsuspension wurden zu 700 µl des Standardpuffers gegeben und zum Starten der Reaktion mit 250 µl einer 200 mM Lösung des Nitrils in Standardpuffer versetzt. Die Konzentration der Zellen in der Zellsuspension war hierbei so bemessen, dass das Nitril nach 10 min bei 20°C zu 5 - 30 % umgesetzt war. Nach 10 min bei 20°C wurde die Reaktion durch Zugabe von 20 µl halbkonzentrierter Phosphorsäure abgestoppt und die Zellen wurden durch Zentrifugation abgetrennt.

| **HPLC-Analytik** | |
|---|---|
| Säule | Intersil ODS-3V |
| Mobile Phase | Gemisch aus 10 mM Kaliumphosphatpuffer pH 2,3 und Acetonitril im Verhältnis 85:15 für Methioninnitril, MHA-Nitril und Aceto-cyanhydrin bzw. 99:1 für alle anderen Substrate |
| Flussrate | 1 ml/min |
| Detektion | UV bei 200 nm |

Die Aktivität von einem Unit (U) wurde definiert als die Menge an Enzym, die 1 µmol N-Formylvalinnitril in einer Minute zum Amid umsetzt. Die spezifische Aktivität wird in U pro mg Biotrockenmasse (U/mg BTM) angegeben.

Diese wird mit dem Moisture Analyser Modell MA45 (Sartorius) gemessen.

### Beispiel 5

### Co-Expression derfür die Nitrilhydrataseα-Untereinheit, die β-Untereinheit und das Protein p15K kodierenden Gene.

Die Expression wurde wie in Beispiel 3 beschrieben mit dem transformierten Stamm E. coli DSM 14459, der das Plasmid pUD15 enthält, durchgeführt. Die spezifische Aktivität der Zellen betrug 23 U/mg BTM.

### Beispiel 6

### Co-Expression der für die Nitrilhydratase α-Untereinheit, die β-Untereinheit, das Protein p15K und das Kobalttransporter kodierenden Gene.

Die Expression wurde wie in Beispiel 3 beschrieben mit dem transformierten Stamm E. coli DSM 14459, der die Plasmide pUD15 und pUD16 enthält, durchgeführt. Die spezifische Aktivität der Zellen betrug 81 U/mg BTM.

### Beispiel 7

### Substratspezifität

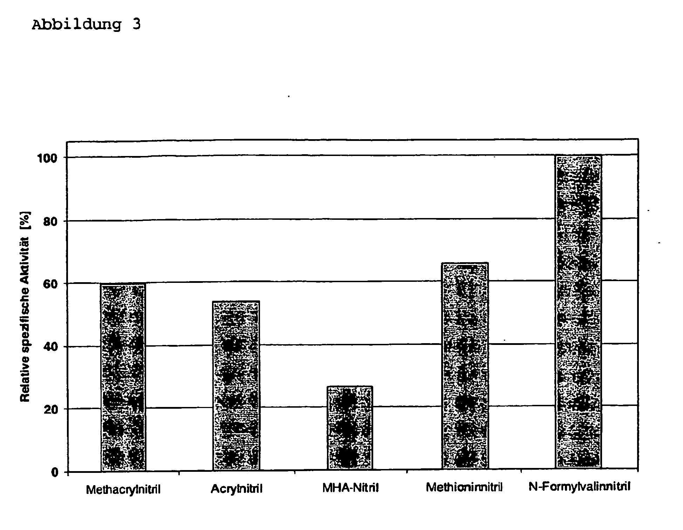

Verschiedene Nitrile wurden analog zu Beispiel 3 mit ruhenden transformierten E. coli DSM 14459 Zellen, die das Plasmid pUD15 enthalten, umgesetzt. Die spezifische Aktivität, die mit N-Formylvalinnitril erhalten wurde, wurde gleich 100 % gesetzt. Die anderen Aktivitäten wurden relativ dazu angegeben. Die Ergebnisse sind in Abbildung 3 wiedergegeben.

### Beispiel 8

### Wachstum von transformiertem E. coli DSM 14459 in Gegenwart von Co²⁺-Salzen

Transformierte E. coli DSM 14459 Zellen, die entweder nur das Plasmid pUD15 oder pUD15 und pUD16 enthalten, wurden wie in Beispiel 3 beschrieben, angezogen. Die Cobaltkonzentration im Medium wurde dabei von 0,5 bis 2 mM variiert. Nach 24 Stunden wurde die optische Dichte der Kultur bei 600nm gemessen.

| | E. coli mit pUD15 | E. coli mit pUD15 und pUD16 |
|---|---|---|
| 0,5 mM CoCl2 | 2,808 | 2,524 |
| 1,0 mM CoCl2 | 2,6955 | 2,173 |
| 2,0 mM CoCl2 | 2,330 | 2,113 |

Es zeigt sich, dass auch bei hohen Cobaltkonzentrationen nur ein geringer Einfluss auf das Wachstum der Zellen festzustellen ist.

### Beispiel 9

### Umsetzung von Methioninnitril mit transformierten E. coli DSM 14459 Ruhezellen, die das Plasmid pUD15 enthalten.

E. coli DSM 14459 Zellen, die das Plasmid pUD15 enthalten, wurden wie in Beispiel 3 beschrieben, angezogen und abzentrifugiert. 2,8 g der Zellen, bezogen auf das Feuchtgewicht, wurden in 47,2 ml 50 mM Kaliumphosphatpuffer pH7,5 resuspendiert und Methioninnitril bei 20°C unter heftigem Rühren kontinuierlich mit einer solchen Rate zugegeben, dass die Konzentration während der Reaktion 15 g/L zu keinem Zeitpunkt überschritt. Der pH wurde konstant bei 7,5 gehalten. Die Reaktionsverfolgung wurde mittels HPLC wie in Beispiel 4 beschrieben, durchgeführt. Nach 320 min waren 9,1 g des Nitrils vollständig zu 10,4 g Amid umgesetzt worden. Das entspricht einer Konzentration von 176 g/L.

### Kurze Beschreibung der Abbildungen

### Abbildung 1

### Plasmid pUD15

- rhaP: Rhamnose-Promotor
- beta: Gen der β-Untereinheit der Nitrilhydratase
- alpha: Gen der α-Untereinheit der Nitrilhydratase
- P15K: Gen des Hilfsproteins P15K
- ori: Replikationsorigin
- bla: Gen für Ampicillin-Resistenz (β-Lactamase)

### Abbildung 2

### Plasmid pUD16

- rhaP: Rhamnose-Promotor
- CoTrans: Gen des Kobalttransporters
- ori: Replikationsorigin
- Cmr: Gen für Chloramphenicol-Resistenz

### Abbildung 3

Relative spezifische Aktivität bei der Umsetzung verschiedener Nitrile im Vergleich zur Aktivität bei der Umsetzung von N-Formylvalinnitril.

## Patentansprüche

1. Isolierter Polynukleotidcluster aus Rhodococcus opacus, enthaltend vier Nukleotidsequenzen, die für vier Polypeptide mit Aminosäuresequenzen kodieren, die zu jeweils mindestens 90 % identisch sind mit dem in den Sequenzen SEQ ID NO:2 bis SEQ ID NO:4 und SEQ ID NO:6 enthaltenen Aminosäuresequenzen, wobei die Polypeptide die Aktivitäten einer Nitrilhydratase, bestehend aus α- und β-Untereinheit, des Hilfsproteins P15K und eines Kobalttransporters besitzen.

2. Polynukleotide gemäss Anspruch 1, ausgewählt aus der Gruppe:
a) Polynukleotid bestehend aus den Positionen 1 bis 708 der Nukleotidsequenz SEQ ID NO:1 oder der dazu komplementären Nukleotidsequenz,
b) Polynukleotid mit einer Nukleotidsequenz, die der Sequenz aus a) im Bereich der Degeneriertheit des genetischen Codes entspricht,
c) Polynukleotid, das mit den komplementären Sequenzen a) oder b) unter stringenten Bedingungen hybridisiert, wobei die stringenten Bedingungen das Waschen auf 0,1x SSC bei einer Temperatur von 50-68°C beinhalten und
d) Polynukleotid mit einer Nukleotidsequenz aus a), b) oder c), die funktionsneutrale Sinnmutationen enthält,
wobei die Polynukleotide für die β-Untereinheit der Nitrilhydratase kodieren.

3. Polynukleotide gemäss Anspruch 1, ausgewählt aus der Gruppe:
a) Polynukleotid bestehend aus den Positionen 710 bis 1327 der Nukleotidsequenz SEQ ID NO:1 oder der dazu komplementären Nukleotidsequenz,
b) Polynukleotid mit einer Nukleotidsequenz, die der Sequenz aus a) im Bereich der Degeneriertheit des genetischen Codes entspricht,
c) Polynukleotid, das mit den komplementären Sequenzen a) oder b) unter stringenten Bedingungen hybridisiert, wobei die stringenten Bedingungen das Waschen auf 0,1x SSC bei einer Temperatur von 50-68°C beinhalten und
d) Polynukleotid mit einer Nukleotidsequenz aus a), b) oder c), die funktionsneutrale Sinnmutationen enthält,
wobei die Polynukleotide für die α-Untereinheit der Nitrilhydratase kodieren.
wobei die Polynukleotide für das Helferprotein P15K kodieren.

4. Polypeptid gemäss Anspruch 1, enthaltend die Aminosäuresequenzen die zu mindestens 90 % identisch sind mit SEQ ID NO:2 und SEQ ID NO:3, wobei das Polypeptid die Aktivität einer Nitrilhydratase aufweist.

5. Sonde oder Primer, enthaltend mindestens 20 aufeinanderfolgende Nukleotide innerhalb der Positionen 1 bis 1327 aus der Nukleotidsequenz SEQ ID NO:1 oder seiner komplementären Form.

6. Isoliertes Polynukleotid gemäss Anspruch 2, das unter stringenten Bedingungen mit dem Komplement mit den Positionen 1 bis 708 aus SEQ ID NO:1 hybridisiert, wobei die stringenten Bedingungen das Waschen auf 0,1 x SSC bei einer Temperatur von 50 bis 68°C beinhalten.

7. Isoliertes Polynukleotid gemäss Anspruch 3, das unter stringenten Bedingungen mit dem Komplement mit den Positionen 710 bis 1327 aus SEQ ID NO:1 hybridisiert, wobei die stringenten Bedingungen das Waschen auf 0,1 x SSC bei einer Temperatur von 50 bis 68°C beinhalten.

8. Vektoren, enthaltend (ein) Polynukleotid(e) ausgewählt aus denen gemäss den Ansprüchen 1 bis 3 und 6 bis 7.

9. Vektor pUD15, bestehend aus der Nukleotidsequenz SEQ ID No:24.

10. Vektor pUD16, bestehend aus der Nukleotidsequenz SEQ ID NO:25.

11. Wirtszelle, transformiert oder transfektiert durch die Einführung eines Polynukleotids gemäss Anspruch 1.

12. Wirtszelle, transformiert durch die Einführung eines Vektors gemäss den Ansprüchen 8 bis 10.

13. Transformierte Wirtszelle gemäß den Ansprüchen 11 oder 12 wobei die Wirtszelle ein Bakterium der Familie Enterobacteriaceae, insbesondere Escherichia ist.

14. Verfahren zur Herstellung von Amiden aus Nitrilen mit Nitrilhydratasen des Ursprungs Rhodococcus oder dieses Enzym enthaltenden Mikroorganismen, bei dem man
a) einen transformierten Mikroorganismus, der überexprimierte Gene mit den Nukleotidsequenzen ausgewählt aus den Ansprüchen 1 bis 3 und 6,7 enthält, in Gegenwart von 0,15 bis 4 mM Co²⁺, unter Bedingungen fermentiert, die zur Bildung der Nitrilhydratase führen,
b) dieses Enzym im Mikroorganismus anreichern lässt, und
c) dieses Enzym aus den Zellen isoliert, oder
d) die Mikroorganismen erntet und als das Enzym enthaltende ruhende Zellen gewinnt, und
e) das Enzym oder die enthaltenen Mikroorganismen mit Verbindungen der allgemeinen Formel 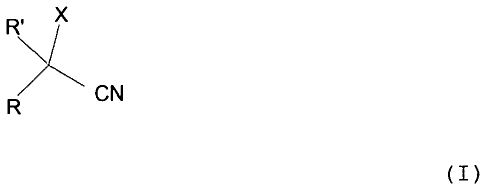 und
R"-CN (II)
in der bedeuten:
X: OH, H, Alkyl mit 1 bis 4 C-Atomen, insbesondere NH₂ ;
R: H, gesättigter Alkylrest mit 1 bis 12 C-Atomen, verzweigt oder unverzweigt, gegebenenfalls NH2-substituiert,
Alkenylreste mit 1 bis 12 C-Atomen, verzweigt oder
unverzweigt, Cycloalkylgruppen mit 3 bis 6 C-Atomen,
mit Alkylthiogruppen substituierte Alkylenreste, wobei Alkyl hier einem C₁ bis C₃-Rest
und Alkylen einem zweiwertigen C₃ bis C₈-Rest entspricht,
R': H, Alkyl mit 1 bis 3 C-Atomen,
R": ein- oder zweikerniger ungesättigter Ring mit 6 bis 12 C-Atomen, gegebenenfalls mit einer oder zwei Alkylgruppen (C1 - C3), Cl, Br, F substituiert, einwertiger Alkylnitrilrest mit 1 bis 6 C-Atomen, zu den entsprechenden Amiden umsetzt.

15. Verfahren gemäss Anspruch 14, **dadurch gekennzeichnet, dass** man Wirtszellen gemäss den Ansprüchen 11 bis 13 einsetzt.

16. Rekombinant erzeugte Nitrilhydratase gemaß Anspruch 14 mit dem Ursprung Rhodococcus opacus, die α-Aminonitrile mit einer spezifischen Aktivität von > 50 U/mg Biotrockenmasse umsetzt.

## Claims

1. Isolated polynucleotide cluster from Rhodococcus opacus which contains four nucleotide sequences which encode four polypeptides which possess amino acid sequences which are in each case at least 90% identical to the amino acid sequences contained in sequences SEQ ID NO:2 to SEQ ID NO:4 and SEQ ID NO:6, with the polypeptides possessing the activities of a nitrile hydratase, composed of an α subunit and a β subunit, of the auxiliary protein P15K and of a cobalt transporter.

2. Polynucleotides according to Claim 1, selected from the group:
a) polynucleotide consisting of positions 1 to 708 in the nucleotide sequence SEQ ID NO:1 or in the nucleotide sequence which is complementary thereto,
b) polynucleotide possessing a nucleotide sequence which corresponds to the sequence from a) within the bounds of the degeneracy of the genetic code,
c) polynucleotide which hybridizes, under stringent conditions, with the complementary sequences a) or b), with the stringent conditions comprising washing to 0.1 x SSC at a temperature of from 50 - 68°C and
d) polynucleotide possessing a nucleotide sequence from a), b) or c) which contains functionally neutral sense mutations,
with the polynucleotides encoding the β subunit of the nitrile hydratase.

3. Polynucleotides according to Claim 1, selected from the group:
a) polynucleotide comprising positions 710 to 1327 in the nucleotide sequence SEQ ID NO:1 or in the nucleotide sequence which is complementary thereto,
b) polynucleotide possessing a nucleotide sequence which corresponds to the sequence from a) within the bounds of the degeneracy of the genetic code,
c) polynucleotide which hybridizes, under stringent conditions, with the complementary sequences a) or b), with the stringent conditions comprising washing to 0.1 x SSC at a temperature of from 50 - 68°C and
d) polynucleotide possessing a nucleotide sequence from a), b) or c) which contains functionally neutral sense mutations,
with the polynucleotides encoding the α subunit of the nitrile hydratase,
with the polynucleotides encoding the auxiliary protein P15K.

4. Polypeptide according to Claim 1, which contains the amino acid sequences which are at least 90% identical to SEQ ID NO:2 and SEQ ID NO:3, with the polypeptide exhibiting the activity of a nitrile hydratase.

5. Probe or primer which contains at least 20 consecutive nucleotides within positions 1 to 1327 from the nucleotide sequence SEQ ID NO:1 or its complementary form.

6. Isolated polynucleotide according to Claim 2 which hybridizes, under stringent conditions, with the complement comprising positions 1 to 708 from SEQ ID NO:1, with the stringent conditions comprising washing to 0.1 x SSC at a temperature of from 50 to 68°C.

7. Isolated polynucleotide according to Claim 3 which hybridizes, under stringent conditions, with the complement comprising positions 710 to 1327 from SEQ ID NO:1, with the stringent conditions comprising washing to 0.1 x SSC at a temperature of from 50 to 68°C.

8. Vectors which contain (a) polynucleotide(s) selected from those according to Claims 1 to 3 and 6 to 7.

9. Vector pUD15, comprising the nucleotide sequence SEQ ID No: 24.

10. Vector pUD16, comprising the nucleotide sequence SEQ ID NO:25.

11. Host cell which is transformed or transfected by introducing a polynucleotide according to Claim 1.

12. Host cell which is transformed by introducing a vector according to Claims 8 to 10.

13. Transformed host cell according to Claim 11 or 12, with the host cell being a bacterium of the family Enterobacteriaceae, in particular Escherichia.

14. Process for preparing amides from nitriles with nitrile hydratases originating from Rhodococcus, or microorganisms comprising this enzyme, in which
a) a transformed microorganism which comprises overexpressed genes having the nucleotide sequences selected from Claims 1 to 3 and 6, 7 is fermented, in the presence of from 0.15 to 4 mM Co²⁺, under conditions which lead to the formation of the nitrile hydratase,
b) this enzyme is allowed to accumulate in the microorganism, and
c) this enzyme is isolated from the cells, or
d) the microorganisms are harvested and isolated as resting cells which comprise the enzyme, and
e) the enzyme or the microorganisms comprised is/are reacted with compounds of the general formula 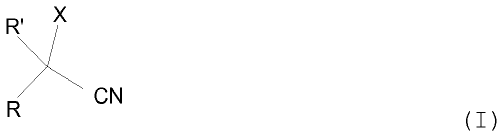 and
R"-CN (II)
in which:
X: is OH, H, alkyl having from 1 to 4 C atoms, or, in particular, NH₂;
R: is H, saturated alkyl radical having from 1 to 12 C atoms, branched or unbranched, optionally NH2-substituted, alkenyl radicals having from 1 to 12 C atoms, branched or unbranched, or cycloalkyl groups having from 3 to 6 C atoms, alkylthio group-substituted alkylene radicals, where alkyl in this case corresponds to a C₁ to C₃ radical and alkylene corresponds to a bivalent C₃ to C₈ radical,
R': is H, or alkyl having from 1 to 3 C atoms,
R": is a mononuclear or binuclear unsaturated ring, which possesses from 6 to 12 C atoms and which is optionally substituted by one or two alkyl groups (C1 - C3) or Cl, Br or F, or a monovalent alkylnitrile radical having from 1 to 6 C atoms,
the corresponding amides.

15. Process according to Claim 14, **characterized in that** host cells according to Claims 11 to 13 are employed.

16. Recombinantly produced nitrile hydratase according to Claim 14, originating from Rhodococcus opacus which converts α-aminonitriles with a specific activity of > 50 U/mg of dry biomass.

## Revendications

1. Batterie isolée de polynucléotides de Rhodococcus opacus, contenant quatre séquences nucléotidiques qui codent pour quatre polypeptides ayant des séquences d'acides aminés dont chacune a une identité d'au moins 90 % avec les séquences d'acides aminés contenues dans les séquences SEQ ID NO:2 à SEQ ID NO:4 et SEQ ID NO:6, les polypeptides possédant l'activité d'une nitrile hydratase, constituée d'une sous-unité α et d'une sous-unité β, celle de la protéine auxiliaire P15K, et celle d'un transporteur de cobalt.

2. Polynucléotides selon la revendication 1, choisis dans le groupe comprenant :
a) le polynucléotide constitué des positions 1 à 708 de la séquence nucléotidique SEQ ID NO:1 ou de la séquence nucléotidique complémentaire de cette dernière,
b) le polynucléotide ayant une séquence nucléotidique qui correspond à la séquence de a) dans la zone de la dégénérescence du code génétique,
c) le polynucléotide qui s'hybride avec les séquences complémentaires a) ou b) dans des conditions stringentes, les conditions stringentes comprenant un lavage sur 0,1x SSC à une température de 50 à 68°C, et
d) le polynucléotide ayant une séquence nucléotidique de a), b) ou c) qui contient des mutations sens fonctionnellement neutres,
les polynucléotides codant pour la sous-unité β de la nitrile hydratase.

3. Polynucléotides selon la revendication 1, choisis dans le groupe comprenant :
a) le polynucléotide constitué des positions 710 à 1327 de la séquence nucléotidique SEQ ID NO:1 ou de la séquence nucléotidique complémentaire de cette dernière,
b) le polynucléotide ayant une séquence nucléotidique qui correspond à la séquence de a) dans la zone de dégénérescence du code génétique,
c) le polynucléotide qui s'hybride aux séquences complémentaires a) ou b) dans des conditions stringentes, les conditions stringentes comprenant un lavage sur 0,1x SSC à une température de 50 à 68°C, et
d) le polypeptide ayant une séquence nucléotidique de a), b) ou c), qui contient des mutations sens fonctionnellement neutres,
les polynucléotides codant pour la sous-unité α de la nitrile hydratase,
les polynucléotides codant pour la protéine auxiliaire P15K.

4. Polypeptide selon la revendication 1, contenant les séquences d'acides aminés ayant une identité d'au moins 90 % avec SEQ ID NO:2 et SEQ ID NO:3, le polypeptide présentant l'activité d'une nitrile hydratase.

5. Sonde ou amorce contenant au moins 20 nucléotides consécutifs à l'intérieur des positions 1 à 1327 de la séquence nucléotidique SEQ ID NO:1 ou de sa forme complémentaire.

6. Polynucléotide isolé selon la revendication 2, qui s'hybride dans des conditions stringentes au complément comportant les positions 1 à 708 de SEQ ID NO:1, les conditions stringentes comprenant un lavage sur 0,1x SSC à une température de 50 à 68°C.

7. Polynucléotide isolé selon la revendication 3, qui s'hybride dans des conditions stringentes au complément comportant les positions 710 à 1327 de SEQ ID NO:1, les conditions stringentes comprenant un lavage sur 0,1x SSC à une température de 50 à 68°C.

8. Vecteurs contenant un ou plusieurs polynucléotides choisis parmi ceux selon les revendications 1 à 3 et 6 à 7.

9. Vecteur pUD15, consistant en la séquence nucléotidique SEQ ID NO:24.

10. Vecteur pUD16, consistant en la séquence nucléotidique SEQ ID NO:25.

11. Cellule hôte, transformée ou transfectée par insertion d'un polynucléotide selon la revendication 1.

12. Cellule hôte, transformée par insertion d'un vecteur selon les revendications 8 à 10.

13. Cellule hôte transformée selon les revendications 11 ou 12, la cellule hôte étant une bactérie de la famille des Enterobacteriaceae, en particulier Escherichia.

14. Procédé de préparation d'amides à partir de nitriles à l'aide de nitrile hydratases provenant de Rhodococcus ou de microorganismes contenant cette enzyme, dans lequel :
a) on fermente un microorganisme transformé, qui contient des gènes surexprimés ayant des séquences nucléotidiques choisies parmi les revendications 1 à 3 et 6, 7, en présence de Co²⁺ 0,15 à 4 mM, dans des conditions conduisant à la formation de la nitrile hydratase,
b) on permet à cette enzyme de s'enrichir dans le microorganisme, et
c) on isole cette enzyme à partir des cellules, ou
d) on récolte les microorganismes, et on les obtient sous forme de cellules au repos contenant l'enzyme, et
e) on fait réagir, pour obtenir les amides correspondants, les microorganismes présents avec des composés de formules générales 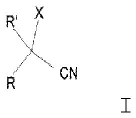 et
R"-CN II
dans lesquelles :
X est OH, H, un radical alkyle ayant 1 à 4 atomes de carbone, en particulier NH₂ ;
R est H, un radical alkyle saturé ayant 1 à 12 atomes de carbone, ramifié ou non ramifié, éventuellement substitué par NH₂ ; des radicaux alcényle ayant 1 à 12 atomes de carbone, ramifiés ou non ramifiés ; des groupes cycloalkyle ayant 3 à 6 atomes de carbone ; des radicaux alkyle substitués par des groupes alkylthio, où alkyle correspond ici à un radical en C₁ à C₃, et alkylène à un radical divalent en C₃ à C₈ ;
R' est H, un radical alkyle ayant 1 à 3 atomes de carbone,
R" est un cycle insaturé, monocyclique ou bicyclique ayant 6 à 12 atomes de carbone, comportant éventuellement un ou deux groupes alkyle en C₁-C₃ substitués par Cl, Br, F ; un radical alkylnitrile monovalent ayant 1 à 6 atomes de carbone.

15. Procédé selon la revendication 14, **caractérisé en ce qu'**on utilise des cellules hôtes selon les revendications 11 à 13.

16. Nitrile hydratase produite par recombinaison selon la revendication 14, provenant de Rhodococcus opacus, qui convertit des α-aminonitriles ayant une activité massique > 50 U/mg de biomasse sèche.
